# EUROPEAN PATENT APPLICATION

(11) **EP 3 305 797 A1**
(43) Date of publication of application: **11.04.2018**
(21) Application number: 16799401.1
(22) Date of filing: 20.05.2016
(51) Int. Cl.: C07H 15/203, A61K 31/7004

(54) **ANTAGONISTS OF NK1 RECEPTORS DERIVED FROM CARBOHYDRATES, PRODUCTION METHOD AND MEDICAL USE**

(30) Priority: 27.05.2015 ES 201530732
(71) Applicant: Consejo Superior de Investigaciones Científicas (CSIC), 41013 Sevilla (ES); Universidad De Sevilla, 41013 Sevilla (ES)
(72) Inventor: KHIAR EL WAHABI, Noureddine, 41092 Sevilla (ES); FERNÁNDEZ FERNÁNDEZ, Inmaculada, 41012 Sevilla (ES); RECIO JIMÉNEZ, Rocío, 41012 Sevilla (ES); LÓPEZ LÁZARO, Miguel, 41012 Sevilla (ES); CALDERON MONTAÑO, José Manuel, 41012 Sevilla (ES)
(74) Representative: Pons Ariño, Angel
(86) International application number: PCT/ES2016/070383
(87) International publication number: WO 2016/189179

(57) **Abstract**

The invention relates to a compound of general formula (I), and to the use thereof in medicine, or for the production of a medicament for the treatment of different diseases, preferably a cancer such as melanoma, lung carcinoma or breast cancer. For this purpose, the invention also relates to a pharmaceutical composition comprising said compound. In addition, the invention relates to a method for producing the compound of general formula (I).

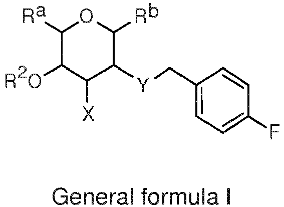

## Description

### Sector of the technique

This invention is primarily directed at the pharmaceutical sector with applications for the prevention and/or treatment of diseases and any type of condition or damage involving substance P (SP), or which, although not it is yet involved, proceeds via the NK1, NK2 and NK3 receptor.

### Prior state of the art

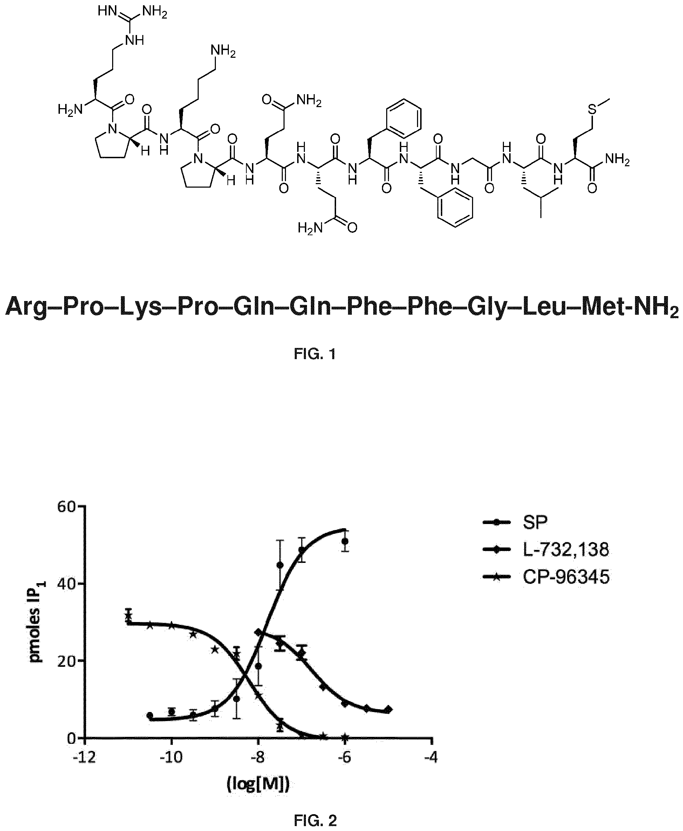

SP (FIG. 1), neurokinin A (NKA) and neurokinin B (NKB) are mammal tachykinins that act as both neurotransmitters and neuromodulators [Nakanishi, S. Annu Rev Neurosci. 1991, 14, 123]. These peptides exercise their biological effects on the central nervous system (CNS) by binding to their receptors bound to G, NK1, NK2 and NK3 proteins [Quatara, L.; Maggi, C. A. Neuropeptides, 1998, 32, 1].

In particular, the NK1 receptor (NK1R), the cognate receptor of SP and the most abundant of the tachykinins in the mammalian CNS, is present in areas of the brain involved in the regulation of affective behaviour and the mediation of anxiety, stress and depression [Leroy, V.; Mauser, P.; Gao, Z.; Peet, N. P. Neurokinin Receptor Antagonists. Expert Opin. Invest. Drugs 2000, 9, 735-746].

The observation that SP release is associated with various psychopathological processes means that the NK1 receptor is currently a highly relevant therapeutic target [(a) Bremer, A.A. and Leeman, S.E. (January 2010) Substance P. In: Encyclopedia of Life Sciences (ELS). John Wiley & Sons, Ltd: Chichester. DOI: 10.1002/9780470015902.a0000206.pub2 (b) Rupniak, N.M.J.; Kramer, M. S. Substance P and related tachykininis. Neuropsychopharmacology. The Fifth Generation of Progress. 2002. Lippincott, Williams, & Wilkins (Ed. Davis, K.L.; Charney, D.; Coyle, J.T.; Nemeroff, C.)].

Consequently, NK1 receptor antagonists are currently being considered as potential therapeutic agents for a large number of pathologies such as migraine, [Moskowitz, M. A. Trends Pharmacol. Sci. 1992, 13, 307-311] rheumatoid arthritis, [Lotz, M.; Carson, D. A.; Vaughan, J. H. Science 1987, 235, 893-895] astha, inflammatory bowel disease, emesis, [Dando, T: M.; Perry, C. M. DRUGS, 2004, 64, 777-794], el cancer [Folkers, K.; Feng, D. M.; Asano, N.; Hakanson, R.; Wiesenfeld-Hallin, Z.; Leander, S. Spantide II, Proc. Natl. Acad. Sci. USA 1990, 87, 4833-4835], as well as central nervous system disorders such as anxiety, Parkinson's disease and depression [Quartara, L.; Altamura, M. Curr. Drug Targets 2006, 7, 975-992].

Recent data indicating that NK1 receptor antagonists exert significant anticancer activity are of particular interest [(a) Muñoz, M.; Rosso, M.; Pérez, A.; Coveñas, R.; Rosso, R.; Zamarriego, C; Piruat, J.I. Neuropeptides, 2005, 39, 427. (b) Muñoz, M.; Pérez, A.; Rosso, M.; Zamarriego, C.; Rosso, R. Melanoma Res. 2004, 14, 183. (c) Esteban, F.; Muñoz, M.; Gonzalez-Moles, M.A.; Rosso, M. Cancer Metastasis Rev. 2006, 25, 137]. In this regard, the anticancer activity of the NK1 receptor antagonists turns out to be very broad spectrum, allowing the treatment of human melanoma, neuroblastoma, human Hodgkin's lymphoma, lymphoblastic leukemia, human rhabdomyosarcoma, human Burkitt's lymphoma, human lung carcinoma, human Edwing's sarcoma, human glioma, human osteosarcoma, malignant human gangliomas, human invasive malignant melanoma, human metastatic melanoma cells and human breast cancer among others [Muñoz, M. Use of non-peptide NK1 receptor antagonists for the production of apoptosis in tumour cells. Patent application number: PCT/ES2005/000068]. In this regard, it is important to highlight that it has recently been shown that the NK1 receptor is highly over-expressed in a large number of aggressive tumours, [(a) Hennig, I. M.; Laissue, J. A.; Horisberger, U.; Reubi, J. C. Int. J. Cancer 1995, 61, 786. (b) Singh, D.; et al. Proc. Natl. Acad. Sci. USA 2000, 97, 388] in particular in glioma, astrocytomas and glioblastomas, [(a) Palma, C.; Maggi, C.A. Life Sci. 2000, 67, 985-1001. (b) Lai, J. P., Douglas, S. D., Wang, Y. J.; Ho, W. Z. Clin. Diagn. Lab. Immunol. 2005, 12, 537-541], where the level of expression correlates with the degree of malignancy [Yamaguchi, K.; Richardson, M. D.; Bigner, D. D.; Kwata, M. M. Cancer Chemother. Pharmacol. 2005, 56, 585].

The first designs of NK1 receptor antagonists based on the structure of SP have given rise to compounds with a peptide structure with low affinities and poor metabolic stabilities [(a) Enberg, G.; Svensson, T. H.; Rosell, S.; Folkers, K. Nature 1981, 293, 222. (b) Folkers, K., Hakanson, R.; Horig, J.; Jie-Cheng, X.; Leander, S.. Br. J. Pharmacol. 1984, 83, 449. (c) Folkers, K.; Feng, D. M.; Asano, N.; Hakanson, R.; Wiesenfeld-Hallin, Z.; Leander, S. Spantide II. Proc. Natl. Acad. Sci. USA 1990, 87, 4833]. The discovery at the start of the 90s in the last century of the first non-peptide antagonist of the NK1 receptors, CP-96345 [Snider, R. M.; Constantine, J. W.; John A. Lowe, J. A.; Kelly P. Longo, K. P.; Lebel, W. S.; Woody, H. A.; Drozda, S. E.; Desai, M. C.; Vinick, F. J. Robin W. Spencer, R. W.; Hess. H.-J. Science 1991, 251, 435] has driven research in this area, not only in the academic field, but also at the industrial level, with nearly all the major pharmaceutical companies working in this field with the aim of identifying selective and potent NK1 R antagonists [(a) Giardina, G. A.; Gagliardi, S.; Martinelli, M. IDrugs, 2003, 6, 758. (b) Huang, S.-C.; Korlipara, V. L. Exp. Pat. Opin. Ther. Pat. 2010, 20, 1019-1045]. More than two decades of extensive synthetic and economic effort has yielded the discovery of a considerable number of structurally diverse NK1 receptor antagonists, although none of them with the desired therapeutic success.

Currently, there is only one NK1 receptor antagonist on the market, Aprepitant (Merck), prescribed for the prevention of chemotherapy-induced nausea and vomiting, [Sankhala, K.K.; Pandya, D.M.; Sarantopoulos, J.; Soefje, S.A.; Giles, F.J.; Chawla, S.P. Exp. Opin. Drug Metbol. Toxicol. 2009, 5, 1607]. The main reason for this is that the exact structure of the NK1 receptor, which belongs to the structurally complex superfamily of G-protein-coupled receptors, [(a) Kobilka, B. Angew. Chem. Int. Ed. 2013, 52, 6380. (b) Lefkowitz, R. J. Angew. Chem. Int. Ed. 2013, 52, 6367] is still unknown. Therefore, the design and synthesis of new non-peptide molecules with a high affinity for the NK1 receptor, and preferably with a chemical structure different from known NK1 receptor antagonists, is an important area in modern medical chemistry.

### Description of the invention

A first aspect of the invention relates to a compound of general formula **I**, 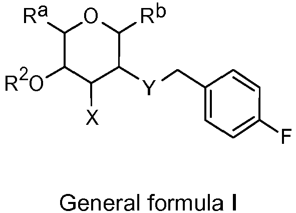 or any of its stereoisomers, or a pharmaceutically acceptable salt thereof, where:
- R^{a} is selected from H and CH₂OR¹, and wherein R¹ is selected from
   hydrogen,
   a C₁-C₂₀ alkyl group,
   a C₆-C₂₀ aryl group,
   a COR^{1a} group, where R^{1a} is independently selected from methyl, *tert-*butyl, and phenyl,
   a group which together with R² forms a cyclic chain and
   a SiR'R"R''' group, where R', R" and R''' are independently selected from methyl, ethyl, *tert*-butyl, and phenyl;
- R^{b} is independently selected from a C₁-C₂₀ alkyl group, a C₆-C₂₀ aryl group, OH and a group of formula II, where Z is selected from O and S, and the carbon adjacent to Z is chiral and can have the *R* or *S* configuration, preferably the *R*; 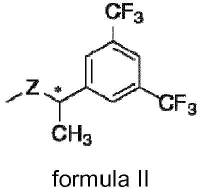
- X is selected from OR³, NR⁴R⁵ and a 3 to 15 members heterocyclic chain, wherein:
   - R³ is selected from H, a C₁-C₂₀ alkyl group, a C₆-C₂₀ aryl group, an acyl group, a alkylsulfonyl group, an arylsulfonyl group, a group of formula III, a group of formula IV, a group of formula V wherein R'''' is a C₁-C₂₀ alkyl group or a C₆-C₂₀ aryl group, and a group that forms a cyclic chain with R²;
   - R⁴ is selected from H, a C₁-C₂₀ alkyl group and a C₆-C₂₀ aryl group;
   - R⁵ is selected from H, a group of formula III, a group of formula IV and a group of formula V wherein R'''' is a C₁-C₂₀ alkyl group or a C₆-C₂₀ aryl group; 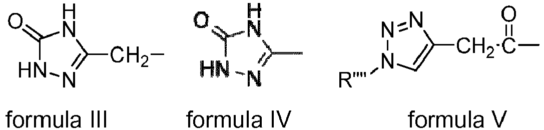
- Y is selected from O, S and NH; and
- R² is H, or forms a cyclic chain with 5 or 6 members along with R¹ or R³, in such a way that in said cyclic chain the O adjacent to R² is separated from the O adjacent to R¹ or R³ by at least one C atom consisting of -C(R⁶)(R⁷)-, and wherein R⁶ and R⁷ are independently selected from H, a C₁-C₂₀ alkyl group and a C₆-C₂₀ aryl group.

The term "alkyl" or "alkyl group", as used in the present invention, refers to aliphatic carbon chains that are linear or branched, saturated or unsaturated, cyclic or heterocyclic and which can have 1 to 50 carbon atoms, preferably these chains have between 1 and 20 carbon atoms, more preferably between 1 and 15, or between 1 and 8, or between 1 and 6, or between 1 and 4 carbon atoms. Examples of alkyl groups as they are used in the invention are, but not limited to, *n*-propyl, isopropyl, *n*-butyl, *sec-*butyl, *n*-pentyl, vinyl, ethynyl, 1- or 2-propenyl, 1- or 2-propynyl, cyclopropyl, cyclohexyl, 4-piperidinyl and also including other preferred groups such as methyl, ethyl, or *tert*-butyl. The alkyl groups can be optionally substituted for one or more substitutes such as, but not limited to, COOH, SO₄H or PO₄H₂.

The term "linear chain" refers in this invention to a chain formed by between 1 and 20 carbon atoms joined together through covalent C-C bonds, its structure being supplemented with hydrogen bonds.

The term "branched-chain" refers in this invention to a carbon chain, in which there is at least 1 additional carbon atom bound to one of the atoms constituting this chain.

The term "saturated chain" refers in this invention to a carbon chain in which there are no double or triple bonds.

The term "unsaturated chain" refers in this invention to carbon chains in which there are at least one double or triple C-C bond.

The term "cyclic chain" refers in this invention to a chain formed by between 3 and 8 carbon atoms with a ring structure, that can be considered the result of eliminating a hydrogen from the terminal carbon in a linear chain and joining it to the first carbon in the chain.

The term "heterocyclic chain" refers in this invention to a monocyclic, bicyclic or tricyclic chain with 3 to 15 members, comprising of carbon atoms and at least one heteroatom selected from the group consisting of nitrogen, oxygen and sulphur and that is unsaturated, saturated or partially saturated. Preferably, the heterocyclic chain has between 4 and 8 members with one or more heteroatoms, more preferably between 5 and 6 members with one or more heteroatoms and even more preferably from 1 to 3 heteroatoms. For the purpose of this invention the heterocyclic can be a monocyclic, bicyclic or tricyclic system that may include fused rings. The nitrogen, carbon and sulfur atoms in the heterocyclic radical can optionally be oxidised; the nitrogen atoms can optionally be quaternised and the heterocyclic radical can be partially or fully saturated or it can be aromatic. Examples of heterocyclics can be, although not limited to: tetrahydrofuran, dioxane, piperidine and 1,2,3-triazole.

The term "aryl", as used in the present invention, refers to aromatic cyclic or aromatic heterocyclic chains of between 6 and 20 carbon atoms. The term "aromatic cyclic chain", as used in the present invention, refers to a carbon chain made up of aromatic monocyclic or polycyclic systems. The term "aromatic heterocyclic chain", as used in the present invention, refers to an aromatic cyclic chain in which one or more atoms in the cyclic chain is a heteroatom selected from N, O and S. The aryl groups are for example, but without limitation, phenyl or naphthyl. Preferably, the aryl group has 6 to 10 carbon atoms and more preferably the aryl group is phenyl. The aryl radicals can be optionally substituted by one or more substituents such as methyl, methoxy, fluoride and trifluoromethyl. Substituent aryl radicals are for example, but without limitation, tolyl or *p*-fluorophenyl.

The term "acyl" refers to a COR^{3a} group wherein R^{3a} can be a C₁-C₂₀ alkyl group or a C₆-C₂₀ aryl group.

The terms "alkylsulfonyl" and "arylsulfonyl" refer to a SO₂R^{3a} group where R^{3a} can be, respectively, a C₁-C₂₀ alkyl group or a C₆-C₂₀ aryl group.

In this patent, the term "pharmaceutically acceptable salt" is to be understood as referring to any salt that when administered to a receptor is capable of providing (directly or indirectly) a compound as described in this document. However, it will be appreciated that pharmaceutically unacceptable salts are also within the scope of the invention, as these may be useful, for example, in the preparation of pharmaceutically acceptable salts. The preparation of salts can be undertaken using methods known in the art.

According to this description, pharmaceutically acceptable salts of the compounds referred to in this document, can be synthesised using conventional chemical methods from an initial compound containing a basic or acidic residue. Generally, these salts are prepared, for example, by reacting the free acid or base forms of the compounds with a stoichiometric quantity of the appropriate base or acid in water or an organic solvent or a mixture of both. Non-aqueous media such as ether, ethyl acetate, ethanol, isopropanol or acetonitrile are generally preferred. Examples of acid addition salts include mineral acid addition salts such as, for example, hydrochloride, hydrobromide, hydroiodide, sulfate, nitrate, phosphate and organic acid addition salts such as, for example, acetate, maleate, fumarate, citrate, oxalate, succinate, tartrate, malate, mandelate, methanesulfonate and p-toluenesulfonate. Examples of base addition salts include inorganic salts such as, for example, sodium, potassium, calcium, ammonium, magnesium, aluminium and lithium salts and salts of organic bases such as, for example, ethylenediamine, ethanolamine, *N, N*-dialkylethanolamine, triethanolamine, glucamine and salts of basic amino acids.

In a preferred embodiment the compound with general formula I that is described in this patent application is a compound wherein R^{b} is a group from formula II, in which Z is selected from O and S.

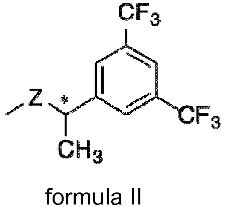

In this group of formula II, the carbon adjacent to Z is chiral and can have configuration *R* or *S*, preferably it is *R,* such that said compound has the following general formula: 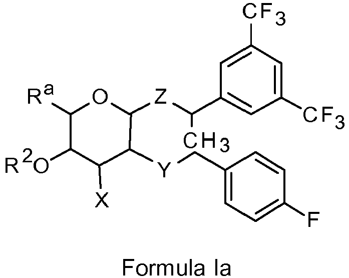 wherein Z, Y, X, R^{a} and R² are as previously defined.

In a still more preferred embodiment, the compound of general formula I of this invention is a compound of formula la wherein Z is O.

In a preferred embodiment, the compound of general formula I or la in any of the described embodiments, is a compound wherein Y is O.

In a preferred embodiment, the compound of general formula I or la in any of the described embodiments, is a compound wherein X is OR³, and R³ has the previously indicated meaning.

In a preferred embodiment, the compound of general formula I or la in any of the described embodiments, is a compound wherein R^{a} is CH₂OR¹ and R² forms a cyclic chain with R¹, wherein the O atom adjacent to R² is separated from the O atom adjacent to R¹ by a -C(R⁶)(R⁷)- group, such that R² forms a cyclic chain with 6 members adjacent to R¹, and wherein R⁶ and R⁷ are independently selected from H, a C₁-C₂₀ alkyl group and a C₆-C₂₀ aryl group. Preferably, the -C(R⁶)(R⁷)- group is a group selected from -C(CH₃)₂- and -CH(C₆H₅)-.

In a preferred embodiment, the compound of general formula I or la in any of the described embodiments, is a compound wherein R^{a} is CH₂OR¹, R¹ and R² form a cyclic chain, and X is OH.

In a preferred embodiment, the compound of general formula I or la in any of the embodiments described in this patent application, is a compound wherein X is OR³ and R² forms a cyclic chain with R³, wherein the O atom adjacent to R² is separated from the O atom adjacent to R³ by a -C(R⁶)(R⁷)- group, such that R² forms a cyclic chain with 5 members with R³, and wherein R⁶ and R⁷ are independently selected from H, a C₁-C₂₀ alkyl group and a C₆-C₂₀ aryl group. Preferably, the -C(R⁶)(R⁷)- group is a group selected from -C(CH₃)₂- and -CH(C₆H₅)-.

In a preferred embodiment, the compound of general formula I or la in any of the embodiments described in this patent application, is a compound wherein X is OR³, R³ and R² form a cyclic chain, in particular a cyclic chain such as has been described in the previous paragraph, and R^{a} can be H, or a CH₂OH group optionally protected by an alcohol protectoing group such as the silyl ethers [for example, *tert*-butyldiphenylsilyl (TDBPS)]. Preferably R^{a} is H.

In a preferred embodiment, the compound of general formula I or la is a compound wherein X is OH and R² is H, where the remaining substituents can have the meaning that is described in any of the embodiments described in this patent application.

In a still more preferred embodiment, the compound of general formula I or la is a compound wherein X is OH, R² is H and R^{a} is selected from H and CH₂OH.

In a preferred embodiment, the compound of general formula I is selected from the group consisting of:

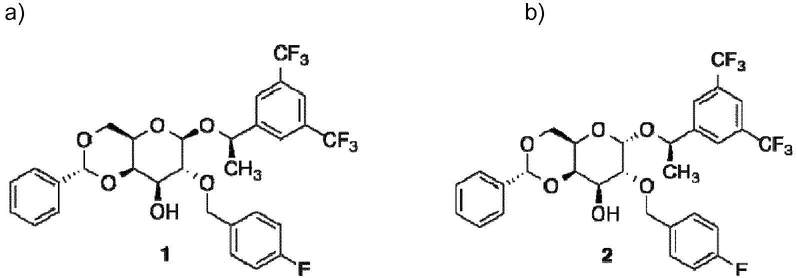

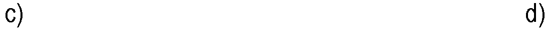

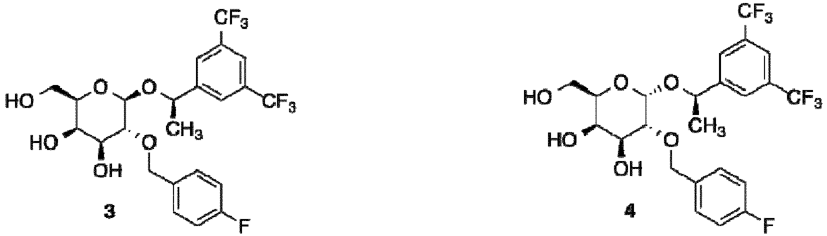

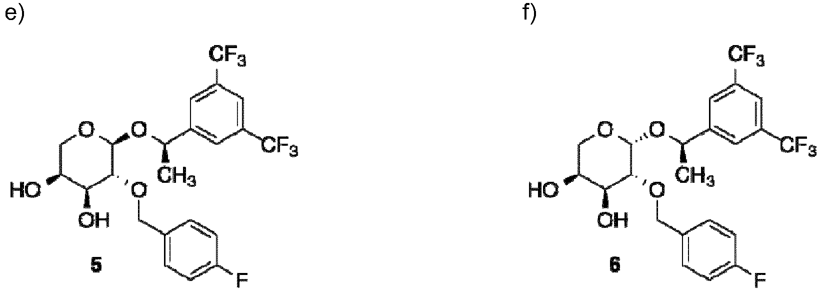

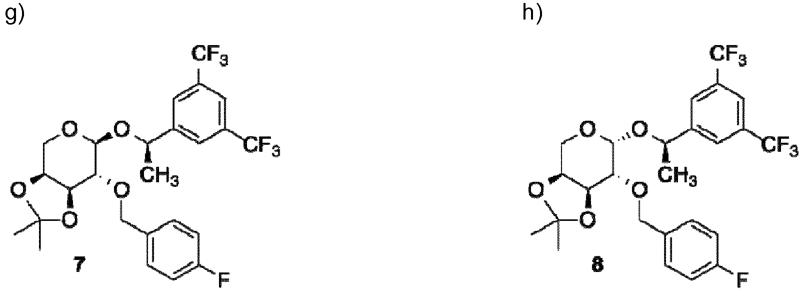

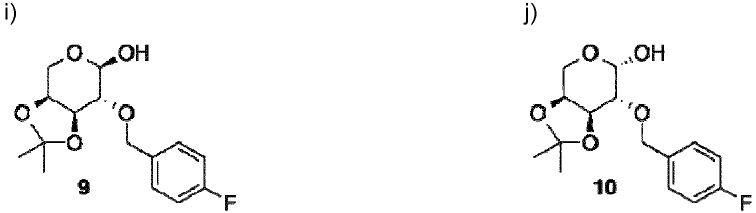

In accordance with the present description, any of the previously defined compounds, that is those compounds that correspond to general formula I, including any of the preferred embodiments or examples, can equally be referred to herein as "compound or compounds of the invention".

The compounds of the invention are analogs of aprepitant and have been shown to exhibit NK1 receptor antagonist behaviour and anticancer activity. Therefore, the compounds of general formula I, and their pharmaceutically acceptable salts, can be useful in medicine to prevent and/or treat different diseases.

A second aspect of the invention relates to a pharmaceutical composition comprising at least one compound of the invention (including the compound of general formula I or any other of its preferred embodiments), or a pharmaceutically acceptable salt thereof, preferably in a therapeutically effective amount. Hereinafter, said pharmaceutical composition can also be referred to as "pharmaceutical composition of the invention".

The composition can, for example, comprise at least one pharmaceutically acceptable adjuvant or vehicle, and/or at least one other pharmaceutically acceptable active ingredient or another excipient known in the field in addition to the compound of general formula I, to give rise to a pharmaceutical composition or medicine that an individual can ingest. The preparation of said pharmaceutical composition can be undertaken using conventional methods known to a person skilled in the art. For their therapeutic application, the compounds of formula I will, preferably, be found in a pharmaceutical composition or a pharmaceutically acceptable or substantially pure form, that is, it has a pharmaceutically acceptable level of purity excluding the normal pharmaceutical additives such as diluents and carriers, and not including material considered toxic at normal dosage levels. The purity levels for the active ingredient are preferably greater than 50%, more preferably greater than 70% and even more preferably greater than 90%. In a preferred embodiment, they are greater than 95% compound of formula I.

The pharmaceutically acceptable adjuvants and vehicles that can be used in said compositions are the adjuvants and vehicles know to those skilled in the art and commonly used in the preparation of therapeutic compositions.

The compounds of formula I described in this invention, as well as the pharmaceutical compositions that contain them can be used along with other additional drugs, or active ingredients, to provide a combination therapy. These additional drugs can be part of the same pharmaceutical composition or, alternatively, they can be provided in the form of a separate composition for simultaneous administration or not with the pharmaceutical composition comprising a compound of formula I.

In another particular embodiment, said pharmaceutical composition is prepared in a solid composition form or in an aqueous suspension, in a pharmaceutically acceptable diluent. The therapeutic composition provided by this invention can be administered by any appropriate administration route, therefore, said composition will be formulated in the pharmaceutical form suitable for the chosen administration route. In a particular embodiment, the administration route of the therapeutic composition provided by this invention is oral, topical, rectal or parenteral (including subcutaneous, intraperitoneal, intradermal, intramuscular, intravenous, etc.).

The amount of the compound of the invention, or of its pharmaceutically acceptable salts, that is therapeutically effective that should be administered (also referred to herein as the therapeutically effective amount), as well as its dosage to treat a pathological condition with these compounds, will depend on numerous factors, including the age and condition of the patient, the severity of the disease, the administration route and frequency, the modulator compound to be used, etc.

Another aspect of the invention relates to the use of at least one compound of the invention, or a pharmaceutically acceptable salt thereof, in any of its embodiments or alternatives, and of the compositions that it comprises, for the manufacture of a pharmaceutical composition or a medicament. It should be understood with regard to the present invention that any of these uses in the field of medicine are also referred to, and analagously, to a compound of general formula I as herein described for its use in medicine, as well as to a method, such as for administration, relating to the compound for the prevention and treatment of diseases. Likewise, this invention includes the use of a compound of general formula I to prepare a composition for its medicinal use, in any of the cases that will be discussed.

The compound of formula I of the invention, as well as the pharmaceutical composition comprising any of these compounds, can be used for the treatment and/or prevention of a disease, such as, and without being limited to, a disease related to the nervous system (preferably alterations or disorders of the central nervous system such as Parkinson's disease, anxiety and depression), rheumatoid arthritis, asthma, inflammatory bowel disease, cancer, post-operative abdominal adhesion, migraine, inflammation, a chronic lung disease, including COPD (chronic obstructive pulmonary disease) and bronchial asthma, obstructive sleep apnea, dysregulation of the cardiac function, arterial thrombosis, osteoporosis, obesity, insulin resistance, Crohn's disease, nausea and vomiting.

The diseases related to the nervous system that can be treated and/or prevented with said compounds or compositions include schizophrenia, Parkinson's disease, stress, anxiety, depression, rabies, psoriasis and pathological pain, such as pain from inflammation and a persistent, degenerative or neuropathic lesion.

The types of cancer that can be treated and/or prevented with said compounds or compositions include melanoma, neuroblastoma, glioma, Hodgkin's lymphoma, lymphoblastic leukaemia, rhabdomyosarcoma, Burkitt's lymphoma, lung carcinoma, Edwing's sarcoma, osteosarcoma, malignant ganglioma, invasive malignant melanoma, metastatic melanoma cells and breast cancer.

Another aspect of the present invention relates to the method for obtaining a compound of general formula I as described, in any of its variants, comprising at least the following steps:
a. Obtaining a thioglycoside of formula VIII, wherein Ra is defined as indicated for a compound of formula I, R is selected from a C₆-C₂₀ aryl group and a C₁-C₂₀ alkyl group, X' is selected from an OH group and an N₃ group, and Y is selected from an OH, S and NH group; 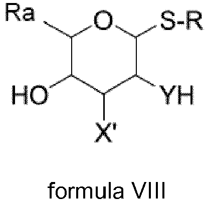
b. Reacting the thioglycoside of formula VIII with alcohol protecting reagents such as, for example, trialkylsilyl halide, dialkyl aryl silyl halide, dialkyl ketones or their dialkylacetals, arylaldehydes or their dialkylacetals or dialkyltin oxide; this reaction can take place in one or several steps, to obtain the compound of formula IX, wherein only the group in position 2 of the carbohydrate is unprotected; 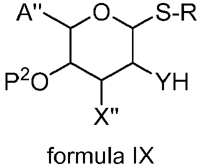 wherein:
   - A" is selected from H and CH₂OP¹, wherein P¹ is selected from
      a C₁-C₂₀ alkyl group,
      a C₆-C₂₀ aryl group,
      a COP^{1a} group, wherein P^{1a} is independently selected from methyl, *tert*-butyl and phenyl,
      a group that forms a cyclic chain along with P², and
      a SiR'R"R''' group wherein R', R" and R''' are independently selected from methyl, ethyl, *tert*-butyl and phenyl,
   - R is selected from a C₆-C₂₀ aryl group and a C1-_{C}20 alkyl,
   - X" is selected from an N₃ group and an OP³ group, wherein P³ is a group that forms a cyclic chain with 5 members with P²,
   - Y is selected from O, S and NH,
   - P² is selected from
      a SiR'R"R''' group wherein R', R" and R''' are independently selected from methyl, ethyl, *tert*-butyl and phenyl,
      a group that forms a cyclic chain with 6 members with P¹, and
      a group that forms a cyclic chain with 5 members with P³,
      so that in the cyclic chain the O adjacent to P² is separated from the O adjacent to P¹ or P³ by a C atom consisting of -C(R⁶)(R⁷)-, and wherein R⁶ and R⁷ are independently selected from H, a C₁-C₂₀ alkyl group and a C₆-C₂₀ aryl group;
c. Reacting the compound of formula IX with an halide or tosylate of *p-*fluorobenzyl to obtain the compound of formula X, wherein A", R, X" and P² are as previously defined, and Y is selected from O, S and NH; 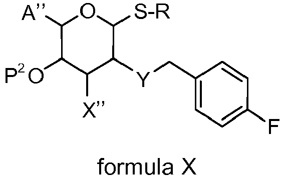
d. Transformation of the compound X obtained in the previous step into a glycosyl donor compound of formula XI, wherein W is selected from OH, an SOR sulfoxide group, an O(OR)₂ phosphite group, and a trichloroacetimidate group; and 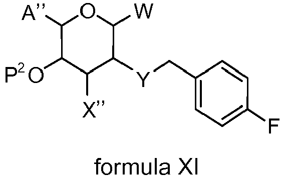
e. Transformation of the compound XI obtained in the previous stage into a compound of general formula I as described in any of the embodiments of this patent application, through a glycosidation reaction.

The transformation of compound XI into the compound of formula I (step e) can be carried out by a process with several steps comprising a glycosidation reaction, preferably but not limited to, through the trichloroacetimidate method, and several protection and deprotection processes on the groups in positions 3, 4, 5 and 6 of the tetrahydropyranyl ring.

In a preferred embodiment, the method for obtaining the compound of general formula I, accord with those embodiments described in this patent application wherein R^{b} is a group of formula II, comprising making compound XI react with 2,2,2-trichloroacetonitrile, in the presence of catalytic quantities of 1,8-diazabicyclo [5.4.0]undec-7-ene (DBU); and subsequently reacting the product obtained with 1-[3,5-bis(trifluoromethyl)phenyl]ethanol and trimethylsilyl trifluoromethanesulfonate.

In other preferred embodiments, the method for obtaining the compound of formula I as described in this patent application additionally comprises the selective protection and/or deprotection of groups in position 3, 4, 5 and/or 6 of the tetrahydropyranyl ring. In other alternative preferred embodiments, the method additionally comprises the reduction and/or alkylation, acylation or Huisgen 1,3-dipolar reaction to obtain compounds of formula I wherein X is NR⁴R⁵ or a heterocyclic chain.

In a preferred embodiment, the method for obtaining the compound of formula I as described in this patent application, also comprises at least the following teps:
f. Reacting the compound obtained in step e of the procedure with an azide-reducing agent, when in the first stage of the procedure a compound of formula VIII is obtained wherein X' is an N₃ group;
g. Reacting the compound obtained in either of steps e or f with tetrabutyl ammonium fluoride in THF;
h. Reacting the compound obtained in either of steps e, f or g with a catalytic quantity of 10-camphorsulfonic acid in methanol;
i. Reacting the compound obtained in either of steps g or h with a dimethoxymethyl derivative with formula CH₃O-C(R⁶)(R⁷)-OCH₃, wherein R⁶ and R⁷ are defined as indicated for the compound of formula I, if in the first stage of the procedure a compound of formula VIII is obtained wherein Ra is CH₂OH;
j. Reacting the compound obtained in either of steps f, g or h with an alkyl halide of formula R⁸-X or an acyl halide of formula R⁹-CO-X, wherein R⁸ is a C₁-C₂₀ alkyl group, a C₆-C₂₀ aryl group, or a group of formula III or formula IV as defined for a compound of formula I, wherein R⁹ is a group of formula V as defined for a compound of formula I.

In a preferred embodiment, obtaining the thioglycoside of formula VIII wherein X' is OH (step a) in the procedure for obtaining the compound of formula I that is described in this patent application comprises:
a.1. Reacting a peracetylated carbohydrate of formula VI, wherein Ra is defined as previously indicated, and Y is selected from an OH group, S and NH; 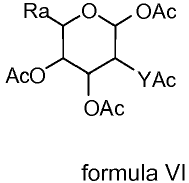 with a thiol with formula RSH, wherein R is selected from a C₆-C₂₀ aryl group and a C₁-C₂₀ alkyl group, in the presence of a Lewis acid type catalyst, such as, for example boron trifluoride-etherate or trimethylsilyl trifluoromethanesulfonate, to obtain a thioglycoside of formula VII, wherein Ra, Y and R have the same meaning as previously established; 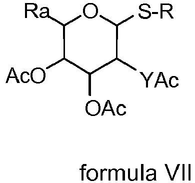
a.2. Obtaining the thioglycoside of formula VIII through a deacetylation reaction on the peracetylated thioglycoside of formula VII with acid or basic catalysis.

This patent application also relates to an alternative method for obtaining the compound of formula I of the invention. This procedure comprises:
a. Obtaining, preferably following steps a.1 and a.2 described above, a thioglycoside of formula VIIIa, wherein Ra is defined as described in relation to a compound of formula I, R is selected from a C₆-C₂₀ aryl group and a C₁-C₂₀ alkyl group, and Y is selected from an OH group, S and NH. 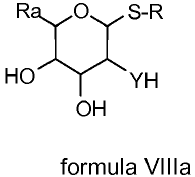
b1. Selectively derivatise the hydroxyl group in position 3 on the ring, in one or several steps. Preferably, the derivatisation comprises the formation of the intermediate dioxanestanylene derivative between the hydroxyls in positions 3 and 4, and its subsequent opening by treatment with an electrophilic reagent, for example, with alkyl halides (chlorides, bromides or iodides), acyl halides or halides or alkyl anhydrides or arylsulfonyl, to obtain a compound of formula XII: 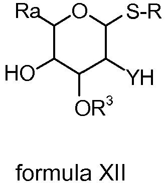 wherein Ra is defined as above, R is selected from a C₁-C₂₀ alkyl group and a C₆-C₂₀ aryl group, R³ is selected from a C₁-C₂₀ alkyl group, a C₆-C₂₀ aryl group, acyl, alkylsulfonyl, arylsulfonyl, a group of formula III, a group of formula IV and a group of formula V.
b2. Reacting the thioglycoside of formula XII with alcohol protecting reagents such as, for example, trialkylsilyl halide, dialkyl aryl silyl halide, dialkyl ketones or their dialkylacetals, arylaldehydes or their dialkylacetals or dialkyltin oxide; this reaction can take place in one or several steps, to obtain the compound of formula XII, wherein only the group in position 2 of the carbohydrate is unprotected; 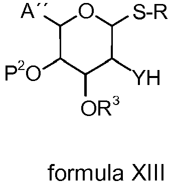 wherein:
   - A" is selected from H and CH₂OP¹, wherein P¹ is selected from
      a C₁-C₂₀ alkyl group,
      a C₆-C₂₀ aryl group,
      a COP^{1a} group, wherein P^{1a} is independently selected from methyl, *tert*-butyl and phenyl,
      a group that forms a cyclic chain along with P², and
      a SiR'R"R''' group wherein R', R" and R''' are independently selected from methyl, ethyl, *tert*-butyl and phenyl,
   - R is selected from a C₆-C₂₀ aryl group and a C₁-C₂₀alkyl,
   - R³ is selected from a C₁-C₂₀ alkyl group, a C₆-C₂₀ aryl group, acyl, alkylsulfonyl, arylsulfonyl, a group of formula III, a group of formula IV and a group of formula V;
   - Y is an O, S or NH group,
   - P² is selected from
      a SiR'R"R''' group wherein R', R" and R''' are independently selected from methyl, ethyl, *tert*-butyl and phenyl, and
      a group that forms a cyclic chain with 6 members with P¹, such that in this chain the O adjacent to P² is separated from the O adjacent to P¹ by a C atom consisting of - C(R⁶)(R⁷)-, and wherein R⁶ and R⁷ are independently selected from H, a C₁-C₂₀ alkyl group, a C₆-C₂₀ aryl group;
c'. Reacting the compound of formula XIII with an halide or tosylate of *p-*fluorobenzyl to obtain the compound of formula XIV, wherein A", R, R³ and P² are as previously defined, and Y is selected from O, S and NH; 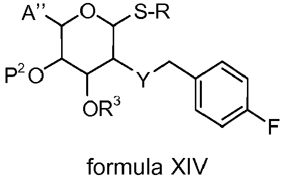
d'. Transformation of the compound XIV obtained in the previous step into a glycosyl donor compound of formula XV, wherein W is selected from OH, an SOR sulfoxide group, an O(OR)₂ phosphite group, and a trichloroacetimidate group; 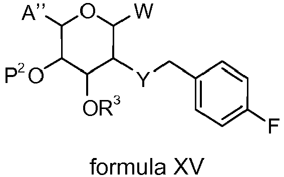
e'. Transformation of the compound XV obtained in the previous step into a compound of general formula I as described in this patent application, through a glycosidation reaction.

The transformation of compound XV into the compound of formula I (step e') can be carried out by a process with several stages comprising a glycosidation reaction, preferably but not limited to, through the trichloroacetimidate method, and several protection and deprotection processes on the groups in positions 3, 4, 5 and 6 of the tetrahydropyranyl ring.

### Brief description of the figures

**FIG 1****.** Amino acid sequence that makes up substance P (SP).
**FIG 2****.** Dose-response curves for NK1 antagonists with reference CP-96345 and L-732,138 determined by inhibition of substance P (SP) through the IP-One assay.
**FIG 3****.** Dose-response curves for derivatives **2**, **4** and **5** determined by inhibition of substance P (SP) through the IP-One assay.
**FIG 4****.** SP activity inhibition data with derivatives **3**, **6** and with reference antagonists CP-96345 and L-732,138 obtained at a ligand concentration of 10⁻⁶M.

### Examples

### EXAMPLE 1. Procedure for obtaining compounds of formula XIV.

### 1.1. Preparation of thioglycosides.

### Phenyl 2,3,4,6-tetra-O-acetyl-1-thio-β-D-galactopyranoside, 12

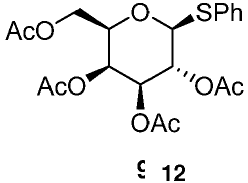

Boron trifluoride etherate (5.00 mL, 40 mmol) is added dropwise to a solution of D-galactose pentaacetate (3.90 g, 10.00 mmol) in anhydrous dichloromethane (40 mL), at 0° °C. After 15 minutes of stirring at room temperature, benzenethiol (1.07 mL, 10.50 mmol) is added. The stirring is maintained at room temperature overnight to continue the reaction; then thin layer chromatography is used to ensure that the starting product has been consumed. Next, a saturated aqueous solution of NaHCO₃ is added, the two phases are separated and the aqueous phase is extracted with CH₂Cl₂ (2x40mL) and the combined organic extracts are washed with saturated NaCl solution. The organic phase is dried over anhydrous Na₂SO₄, filtered and evaporated in vacuo. The residue obtained has a high degree of purity although it is purified by column chromatography, using ethyl acetate-hexane in a ratio of 1:4 as eluent, yielding product 12 (3.96 g, 9.00 mmol) as a white solid with a yield of 90%. P.f.: 115-116 °C.¹H-NMR (500MHz, CDCl₃): δ 7.52-7.5 (m, 2H), 7.32-7.31 (m, 3H), 5.42 (d, *J*=2.7 Hz, 1 H), 5.24 (t, *J*=10.0 Hz, 1 H), 5.05 (dd, *J*=3.3 and 9.9 Hz, 1 H), 4.72 (d, *J*=, 10.0 Hz, 1 H), 4.19 (dd, *J*=6.9 and 11.4 Hz, 1H), 4.12 (dd, *J*=6.2 and 11.3 Hz, 1H), 3.94 (t, *J*=6.9 Hz, 1H), 2.12 (s, 3H), 2.10 (s, 3H), 2.04 (s, 3H), 1.97 (s, 3H). ¹³C NMR (500 MHz, CDCl₃) δ 170.5, 170.4, 170.2, 169.6, 132.7, 132.6, 129.0, 128.3, 86.8, 72.1, 67.3, 67.4, 61.8, 21.0, 20.8. HRMS: calculated for C₂₀H₂₅O₉S: [M+H]⁺ 441.1219 found 441.1200 (-4.4 ppm).

### Phenyl 2,3,4-tri-O-acetyl-1-thio-α-L-arabinopyranoside, 13.

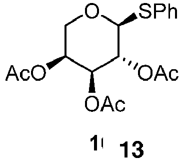

The synthesis follows a procedure similar to that described for the preparation of **12**, starting from β-L-arabinose tetraacetate (5.24 g, 16.48 mmol), boron trifluoride etherate (8.27 mL, 65.90 mmol) and benzenethiol (1.77 mL, 17.30 mmol), to obtain product **10** (6.00 g, 16.30 mmol) as an orange oil with quantitative yield, which is used directly in the next reaction without prior purification. ¹H-NMR (500MHz, CDCl₃): δ 7.49-7.47 (m, 2H), 7.29-7.24 (m, 3H), 5.26-5.24 (m, 1 H), 5.22 (d, *J*=8.1 Hz, 1 H), 5.11 (dd, *J*=3.4 and 8.5 Hz, 1H), 4.82 (d, *J*=, 7.9 Hz, 1H), 4.10 (dd, *J*=3.6 and 12.3 Hz, 1H), 3.65 (dd, *J*=1.9 and 12.7 Hz, 1H), 2.05 (s, 3H), 2.04 (s, 3H), 1.98 (s, 3H). ¹³C NMR (500 MHz, CDCl₃) δ 169.8, 169.5, 169.0, 132.0, 129.1, 128.8, 128.7, 127.7, 125.2, 86.3, 70.3, 68.3, 67.4, 65.1, 20.6, 20.5, 20.3. [α]²⁰_{D}: +24.70 (c 1, chloroform).

### 1.2. Zemplen deacetylation for the synthesis of polyhydroxy derivatives.

### Phenyl 1-thio-β-D-galactopyranoside, 14

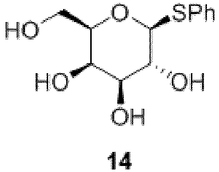

A solution of 1 M sodium methoxide in methanol (36 mL, 3.60 mmol) is added to a solution of compound **12** (3.96 g, 9.00 mmol) in methanol (45 mL). After stirring for 30 min, the reaction is neutralised with acidic resin and filtered to obtain product **14** (2.40 g, 8.82 mmol) as a white solid with a quantitative yield, which is used directly in the next reaction without the need for purification. P.f.: 114-115 °C. ¹H-NMR (500MHz, MeOD): δ 7.56-7.54 (m, 2H), 7.30-7.27 (m, 2H), 7.24-7.21 (m, 1 H), 4.59 (d, *J*=9.8 Hz, 1H), 3.90 (d, *J*=3.0 Hz, 1H), 3.78-3.74 (m, 1H), 3.72-3.69 (m, 1H), 3.61 (t, *J*=9.5 Hz, 1 H), 3.57 (t, *J*=6.1 Hz, 1 H), 3.50 (dd, *J*=3.3 and 9.2 Hz, 1 H). ¹³C NMR (500 MHz, MeOD) δ 136.1, 132.2, 129.8, 128.0, 90.3, 80.6, 76.4, 71.0, 70.4, 62.6. HRMS: calculated for C₁₂H₁₆O₅NaS: [M+Na]⁺ 295.0616 found 295.0605 (3.6 ppm).

### Phenyl 1-thio-α-L-arabinopyranoside, 15.

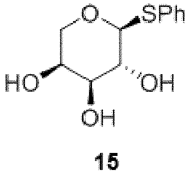

The synthesis follows a procedure similar to that described for the preparation of **14**, starting from **13** (6.00 g, 16.30 mmol) and sodium methoxide in methanol 1M (10.00 mL, 10.00 mmol), giving **15** (3.91 g, 16.15 mmol) as a red solid with a quantitative yield, which is used directly in the next reaction without prior purification. P.f.: 114-115 °C. ¹H-NMRM (500MHz, MeOD): δ 7.53-7.50 (m, 2H), 7.31-7.22 (m, 3H), 4.64 (d, *J*=8.1 Hz, 1H), 3.98 (dd, *J*=3.6 and 12.2 Hz, 1H), 3.89 (td, *J*=1.9 and 3,4 Hz, 1H), 3.70 (t, *J*=8.2 Hz, 1H), 3.60-6.56 (m, 2H). ¹³C NMR (500 MHz, MeOD) δ 136.1, 132.4, 129.8, 128.1, 90.4, 74.9, 71.6, 69.5 (2). [α]²⁰_{D}: +15.05 (c 1, chloroform).

### 1.3. Preparation of 3,4-O-isopropylidene acetals.

### Phenil 3,4-O-isopropylidene-1-thio-β-D-galactopyranoside, 16.

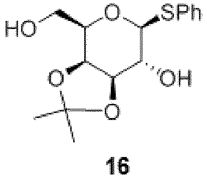

A catalytic amount of 10-camphorsulfonic acid (CSA) (60.35 mg, 0.26 mmol) it added to a suspension of polyhydroxy derivative **14** (2.21 g, 8.12 mmol) in 60 mL of 2,2-dimethoxypropane (2,2-DMP), at room temperature and under an argon atmosphere, and the mixture is left stirring for 48 hours. After that time, the reaction is neutralised with triethylamine, filtered to remove the ammonium salt that has formed and the solvent is evaporated in vacuo. The residue obtained is dissolved in the minimum possible amount of toluene and evaporated in vacuo, after repeating this process twice the mixed acetal is obtained, together with a small amount of the desired diol. The crude mixture is dissolved in the minimum possible amount of methanol, treated with a catalytic amount of CSA (60.35 mg, 0.26 mmol) at 0°°C and stirred at room temperature for 5 minutes. It is then neutralised with triethylamine, the ammonium salt that has formed is filtered off and the solvent is evaporated in vacuo. The residue obtained is dissolved in toluene and evaporated, repeating this process twice, to obtain **16** (2.16 g, 6.92 mmol) as a white solid with an 85% yield. P.f.: 92-93 °C. 1H-NMR (500MHz, CDCl3): δ 7.48-7.46 (m, 2H), 7.25-7.18 (m, 3H), 4.45 (d, *J*=10.0 Hz, 1H), 4.08-4.03 (m, 2H), 3.91-3.87 (m, 1H), 3.81-3.78 (m, 1H), 3.76-3.72 (m, 1H), 3.58 (bs, 1H), 3.55-3.51 (m, 1H), 3.17 (bs, 1H), 1.35 (s, 3H), 1.26 (s, 3H). ¹³C NMR (500 MHz, CDCl₃) δ 132.5, 131.7, 128.8, 127.5, 110.0, 87.1, 79.3, 76.9, 73.6, 71.2, 62.0, 27.8, 26.1. HRMS: calculated for C₁₅H₂₁O₅S: [M+H]⁺ 313.1110 found 313.1107 (-0.9 ppm).

### Phenyl 3,4-O-isopropylidene-1-thio-α-L-arabinopyranoside, 17.

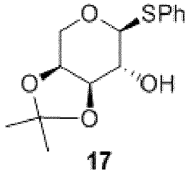

The synthesis follows a procedure similar to that described for the preparation of **16**, starting with **15** (3.91 g, 16.15 mmol), 2,2-dimethoxypropane (120 mL, 969.00 mmol) and 10-camphorsulfonic acid (0.26 mg, 1.13 mmol), yielding product **17** (3.85 g, 13.63 mmol) as a white solid with a yield of 85%. P.f.: 92-93 °C. ¹H-NMR (500MHz, CDCl₃): δ 7.56-7.53 (m, 2H), 7.33-7.28 (m, 3H), 4.53 (d, *J*=9.2 Hz, 1 H), 4.28-4.24 (m, 2H), 4.14-4.19 (m, 1 H), 3.82-3.78 (m, 1 H), 3.67-3.36 (m, 1 H), 1.46 (s, 3H), 1.36 (s, 3H).¹³C NMR (500 MHz, CDCl₃) δ 132.8, 129.1, 128.2, 110.3, 88.3, 78.3, 73.0, 71.7, 65.8, 28.0. [α]²⁰_{D}: +17.56 (c 1, chloroform).

### 1.4. Selective protection of the primary hydroxyl.

### Phenyl 3,4-O-isopropylidene-6-O-tert-butyldiphenylsilyl-1-thio-β-D-galactopyranoside, 18

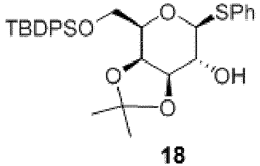

*Tert*-butyldiphenylsilane chloride (TBDPSCI) (0.11 mL, 0.41 mmol) and imidazole (56.34 mg, 0.83 mmol) are added to a solution of **16** (103.10 mg, 0.33 mmol) in DMF (66.20 mL, 2 mL / mmol *13*). After stirring for 5 hours, the reaction is diluted with ethyl acetate (20 mL), neutralised with saturated NH₄Cl solution and the aqueous phase extracted with *n*-pentane (3x20 mL). The organic extracts are dried over anhydrous sodium sulfate and the solvent is evaporated in vacuo. The residue obtained is purified by column chromatography, using ethyl acetate-hexane in a ratio of 1:4. Product **18** (174.49 mg, 0.32 mmol) is obtained as a white solid with a yield of 96%. P.f.: 50 °C. 1H-NM^{R} (500MHz, CDCl₃): δ 7.73-7.70 (m, 4H), 7.54-7.53 (m, 2H), 7.45-7.35 (m, 6H), 7.28-7.27 (m, 3H), 4.44 (d, *J*=10.3 Hz, 1H), 4.28 (dd, *J*=1.9 and 5.4 Hz, 1H), 4.08 (t, *J*=6.2 Hz, 1 H), 4.00-3.89 (m, 3H), 3.55 (ddd, *J*=2.2, 7.1 and 10.1 Hz, 1 H), 1.41 (s, 3H), 1.33 (s, 3H), 1.06 (s, 9H). ¹³C NMR (500 MHz, CDCl₃) δ 135.8, 134.9, 133.5, 133.4, 132.6, 132.4, 129.9, 129.2, 128.1, 127.9, 127.8, 110.3, 88.5, 79.1, 76.9, 73.4, 71.7, 63.1, 28.3, 26.9, 26.7, 26.4, 19.4. HRMS: calculated for C₃₁H₃₈O₅SSiNa: [M+Na]⁺ 573.2107 found 573.2123 (2.8 ppm).

### 1.5. Reaction of the hydroxyl in position 2 with p-fluorobenzyl derivatives.

### Phenyl 2-O-(p-fluorobenzyl)-3,4-O-isopropylidene-6-O-tert-butyldiphenylsilyl-1-thio-β-D-galactopyranoside, 19

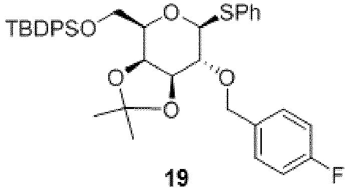

A solution of sodium hydride (0.73 g, 18.21 mmol) in THF (10 mL) is added to a solution of **18** (4.00 g, 6.07 mmol) in THF (80 mL) and left stirring for 1 hour. After that time, IN(Bu)₄ (0.90 g, 2.43 mmol) is added and after 30 minutes stirring a solution of *p-*fluorobenzyl chloride (1.10 mL, 9.11 mmol) in THF (5 mL) is added. The reaction is left stirring for 48 hours and subsequently neutralised with a saturated aqueous solution of ammonium chloride and the aqueous phase is extracted with ethyl acetate (3x40 mL). The organic extracts are washed with saturated sodium chloride solution and dried over anhydrous sodium sulfate. The residue obtained is purified by column chromatography using ethyl acetate-hexane in a ratio or 1:8, to obtain **19** (3.60 g, 5.46 mmol) as a yellow oil with a yield of 90%. ¹H-NMR (500MHz, CDCl₃): δ 7.78-7.74 (m, 4H), 7.58-7.56 (m, 2H), 7.48-7.40 (m, 9H), 7.29-7.25 (m, 2H), 7.10-7.04 (m, 2H), 4.84 (d, *J*=11.3 Hz, 1 H), 4.69 (d, *J*=11.3 Hz, 1 H), 4.65 (d, *J*=9.7 Hz, 1 H), 4.34 (dd, *J*=2.0 and 5.6 Hz, 1 H), 4.28 (t, *J*=6.0 Hz, 1 H), 4.03-3.98 (m, 2H), 3.92 (td, *J*=1.9 and 6.5 Hz, 1 H), 3.55 (dd, *J*=6.4 and 9.7 Hz, 1H), 1.44 (s, 3H), 1.39 (s, 3H), 1.12 (bs, 9H). ¹³C NMR (500 MHz, CDCl₃) δ 162.5, (d, *J*_{CF}=244.1 Hz), 135.8, 135.7, 135.4, 134.9, 134.0, 133.9, (d, *J*_{CF}=3.1 Hz), 133.5 (2C), 131.8, 130.1, (d, *J*_{CF}=8.4 Hz), 129.8, 129.7, 129.0, 127.8 (2C), 127.4, 115.2 (d, *J*_{CF}=21.4 Hz), 110.0, 86.6, 79.8, 78.4, 76.9, 73.6, 72.8, 63.1, 28.0, 26.9, 26.7, 26.4, 19.3. HRMS calculated for C₃₈H₄₃O₅NaSFSi: [M+Na]⁺ 681.2482 found 681.2479 (-0.5 ppm).

### Phenyl 2-O-(p-fluorobenzyl)-3,4-O-isopropylidene-1-thio-α-L-arabinopyranoside, 20.

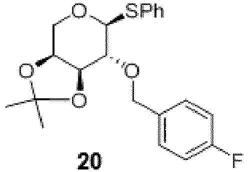

The synthesis follows a procedure similar to that described for the preparation of **19**, starting with **17** (3.85 g, 13.63 mmol), sodium hydride (1.00 g, 41.50 mmol), tetrabutylammonium iodide (2.00 g, 5.45 mmol) and *p*-fluorobenzyl chloride (2.43 mL, 20.45 mmol), yielding product **20** (4.60 g, 12.40 mmol) as a yellow oil with a yield of 91%. ¹H-NMR (500MHz, CDCl₃): δ 7.52-7.50 (m, 2H), 7.39-7.36 (m, 2H), 7.31-7.24 (m, 3H), 7.05-7.00 (m, 2H), 4.79 (d, *J*=8.2 Hz, 1H), 4.78 (d, *J*=11.4 Hz, 1H), 4.31-4.28 (m, 1H), 4.23 (t, *J*=6.1 Hz, 1H), 4.20 (dd, *J*=3.8 and 13.2 Hz, 1H), 3.77 (dd, *J*=3.8 and 13.0 Hz, 1H), 3.59 (dd, *J*=6.1 and 8.0 Hz, 1H), 1.47 (s, 3H), 1.37 (s, 3H). ¹³C NMR (500 MHz, CDCl₃) δ 162.6 (d, *J*_{CF}=245.7 Hz), 131.1, 133.7 (d, *J*_{CF}=3.1 Hz), 132.0, 130.1 (d, *J*_{CF}=8.1 Hz), 129.0, 127.6, 115.3 (d, *J*_{CF}=21.4 Hz), 110.1, 86.5, 78.4, 72.8, 72.7, 64.9, 27.9, 26.3. [α]²⁰_{D}: -9.95 (c 1, chloroform).

### EXAMPLE 2. Glucosidation

### 2.1. Selective deprotection of the anomeric position.

### 2-O-(p-fluorobenzyl)-3,4-O-isopropylidene-6-O-tert-butyldiphenylsilyl-α,β-D-galactopyranose, 21

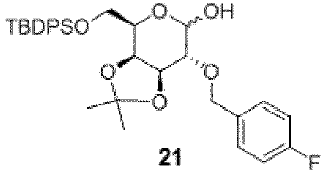

N-bromosuccinimide (NBS) (1.03 g, 5.78 mmol) is added to a solution of **19** (3.02 g, 4.59 mmol) in acetone (120 mL) at 99%, in the absence of light, at -15° °C and stirring continued for 30 minutes. After that time, the reaction is treated with saturated aqueous NaHCO₃ solution and the aqueous phase is extracted with dichloromethane (3x40mL). The organic extracts are dried over anhydrous sodium sulfate and the solvent is evaporated in vacuo. The residue obtained is purified by column chromatography using ethyl acetate-hexane in a 1:8, ratio yielding a yellow syrup, product **21** (2.37g, 4.18 mmol) as a mixture of the two anomers α:β in a ratio of 2:1 and with a yield of 91%. ¹H-NMR (500MHz, CDCl₃): δ 7.71-7.66 (m, 4Ha, 4Hb), 7.43-7.31 (m, 8Ha, 8Hb), 7.05-7.00 (m, 2Ha, 2Hb), 5.15 (dd, *J*=3.7 and 5.0 Hz, 1Ha), 4.78-4.73 (m, 1Ha, 1Hb), 4.67-4.64 (m, 1Ha, 1Hb), 4.41-4.28 (m, 2Ha, 2Hb), 4.23 (t, *J*=6.1 Hz, 1Hb), 3.96-3.80 (m, 2Ha, 2Hb), 3.55 (dd, *J*=7.2 and 5.9 Hz, 1Ha), 3.37 (t, *J*=6.5 Hz, 1Hb), 2.98 (d, *J*=6.4 Hz, 1 Hb), 2.92 (d, *J*=4.1 Hz, 1Ha), 1.41 (s, 3Hb), 1.40 (s, 3Ha), 1.36 (s, 3Ha, 3Hb), 1.05 (s, 9Ha, 9Hb). ¹³C NMR (500 MHz, CDCl₃) δ 162.7, (d, *J*_{CF}=245.6 Hz), 135.8, 135.7 (2C), 133.9, (d, *J*_{CF}=3.5 Hz), 133.8, 133.7, 133.6, (d, *J*_{CF}=3.2 Hz), 133.6, 133.4, 130.0, (d, *J*_{CF}=8.2 Hz), 129.9, (d, *J*_{CF}=8.5 Hz), 129.8 (2C), 127.8, 127.7 (2C), 115.5 (d, *J*_{CF}=21.3 Hz), 115.3, (d, *J*_{CF}=21.1 Hz), 110.0, 109.4, 96.2, 91.0, 80.0, 78.0, 76.3, 74.7, 73.2, 72.9, 72.7, 72.5, 72.1, 68.5, 62.9, 62.8, 27.8, 27.7, 26.9, 26.0, 19.4. HRMS: calculated for C₃₂H₃₉O₆NaSiF: [M+Na]⁺ 589.2398 found 589.2398 (0.1 ppm).

### 2-O-(p-fluorobenzyl)-3,4-O-isopropylidene-α,β-L-arabinopyranoside, 22.

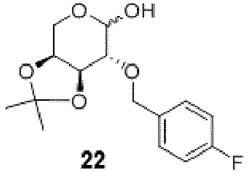

The synthesis follows a procedure similar to that described for the preparation of **21**, starting with **17** (4.60 g, 12.40 mmol) and NBS (8.50 g, 47.23 mmol), yielding product **22** (3.16 g, 11.30 mmol) as a mixture of both α and β anomers, in a 2:1 ratio as a yellow syrup with a 91% yield. ¹H-NMR (500MHz, CDCl₃): δ 7.32-7.26 (m, 2Ha, 2Hb), 6.98-6.94 (m, 2Ha, 2Hb), 5.12 (d, *J*=2.5 Hz, 1Ha), 4.72-4.69 (m, 1Ha, 2Hb), 4.63-4.60 (m, 1Ha, 1Hb), 4.32 (t, *J*=6.3 Hz, 1Ha), 4.17-4.10 (m, 2Ha, 3Hb), 3.82 (d, *J*=13.2 Hz, 1Ha), 3.73 (dd, *J*=2.9 and 13.2 Hz, 1Hb), 3.49 (dd, *J*=3.3 and 6.7 Hz, 1Ha), 3.37 (t, *J*=6.5 Hz, 1Hb), 1.39 (s, 3Hb), 1.38 (s, 3Ha), 1.30 (s, 3Ha, 3Hb). ¹³C NMR (500 MHz, CDCl₃) δ 162.5, (d, *J*_{CF}=245.6 Hz), 162.4 (d, *J*_{CF}=244.8 Hz), 134.0 (d, *J*_{CF}=2.9 Hz), 133.7 (d, *J*_{CF}=2.9 Hz), 129.9 (d, *J*_{CF}=9.4 Hz), 129.8 (d, *J*_{CF}=8.3 Hz), 115.3, (d, *J*_{CF}=21.1 Hz), 115.1, (d, *J*_{CF}=19.8 Hz), 109.9, 109.0, 96.0, 90.9, 80.1, 77.8, 76.4, 74.8, 73.1, 73.0, 72.4, 71.7, 62.7, 60.4, 27.8, 27.7, 26.0, 25.7.

### 2.2. Formation of trichloroacetimidate.

### 2-O-(p-fluorobenzyl)-3,4-O-isopropylidene-6-O-tert-butyldiphenylsilyl-α,β-D-galactopyranoside-trichloroacetimidate, 23

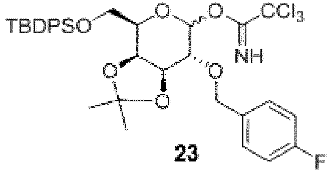

A mixture of 98% 2,2,2-trichloroacetonitrile (0.96 mL, 9.35 mmol) and catalytic amounts of 1,8-Diazabicyclo[5.4.0]undec-7-ene (DBU) at 98% (0.23 mL, 1.50 mmol) is added to a solution of **21** (2.12g, 3.74 mmol) in a mixture of cyclohexane/dichloromethane (50 mL) in a proportion of 4:1 as a solvent. The reaction is left stirring overnight. After that time, water is added, the organic phase is washed with saturated aqueous sodium chloride solution, dried over anhydrous sodium sulfate and evaporated in vacuo. A high purity yellow syrup **23** (2.58 g, 3.63 mmol) is obtained as a mixture of the two α:β anomers in a 3:1 ratio and with an 97% yield. The product obtained is used immediately in the following reaction.

### 2-O-(p-fluorobenzyl)-3,4-O-isopropylidene-α,β-L-arabinopyranoside-trichloroacetimidate, 24.

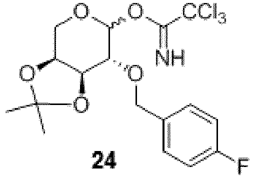

It is synthesised using a procedure similar to that described for the preparation of **23**, starting from **22** (3.16 g, 11.20 mmol), 2,2,2-trichloroacetonitrile at 98% (2.90 mL, 28.25 mmol) and DBU 98% (0.70 mL, 4.52 mmol), yielding product **24** (4.85 g, 11.00 mmol) as a high purity yellow oil as a mixture of both α and β anomers with a 97% yield, which is used immediately in the following reaction.

### 2.2. Glycosidation of trichloroacetimidate.

### Derived from galactose 25 and 26:

Trimethylsilyl trifluoromethanesulfonate (2.8 mL, 0.015 mmol) is added to a solution of **23** (0.16g, 0.22 mmol) and (1*R*)-1-[3,5-bis(trifluoromethyl)phenyl]ethanol (0.17g, 0.67 mmol) in ether (7 mL), under an argon atmosphere, with 200 mg of molecular sieve (4Å) at 0 °C. The reaction is stirred for one hour at room temperature. After that time, NaHCO₃ is added, the solution is filtered through celite and the solvent evaporated in vacuo. A yellow syrup is obtained as a mixture of the two α:β anomers in a ratio of 3:1. Purification by column chromatography using ethyl acetate-hexane in a ratio of 1:15 yields the α anomer (106.51 mg, 0.13 mmol) as the major product with a yield of 60% and the minority β anomer (48.41 mg, 0.06 mmol) with a yield of 25 %.

### α: anomer of (R)-{1-[3,5-bis-(trifluoromethyl)phenyl]ethyl} 2-O-p-fluorobenzyl-3,4-O-isopropylidene-6-O-tert-butyldiphenylsilyl-α-D-galactopyranoside, 25

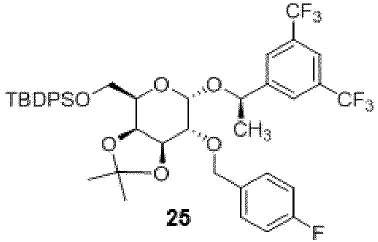

¹H-NMR (500MHz, CDCl₃): δ 7.86 (bs, 2H), 7.82 (bs, 1H), 7.73-7.70 (m, 4H), 7.47-7.37 (m, 6H), 7.21-7.18 (m, 2H), 6.97-6.92 (m, 2H), 4.91 (c, *J*=6.6 Hz, 1H), 4.61 (d, *J*=3.1 Hz, 2H), 4.58 (d, *J*=3.6 Hz, 1 H), 4.42 (dd, *J*=5.5 and 7.8 Hz, 1 H), 4.29 (dd, *J*=2.5 and 5.5 Hz, 1H), 4.18 (td, *J*=2.4 and 6.4 Hz, 1H), 3.98-3.89 (m, 2H), 3.42 (dd, *J*=3.7 and 7.8 Hz, 1H), 1.49 (d, *J*=6.6 Hz, 3H), 1.33 (s, 3H), 1.32 (s, 3H), 1.10 (s, 9H). ¹³C NMR (500 MHz, CDCl₃) δ 162.5, (d, *J*_{CF}=244.8 Hz), 145.8, 135.8 (2C), 133.9, (d, *J*_{CF}=3.2 Hz), 133.7, 133.6, 132.1, (c, *J*_{CF}=33.5 Hz), 129.9, 129.6, (d, *J*_{CF}=8.3 Hz), 127.9, 127.8, 126.9 (d, *J*_{CF}=2.4 Hz), 123.5 (c, *J*_{CF}=272.9 Hz), 121.8, 115.3 (d, *J*_{CF}=21.4 Hz), 109.3, 94.9, 76.2, 76.1, 73.5, 72.6, 71.5, 68.9, 63.3, 28.3, 27.0, 26.5, 24.4, 22.8, 19.4. HRMS: calculated for C₄₂H₄₅O₆F₇SiNa: [M+Na]⁺ 829.2771 found 829.2811 (4.1 ppm).

### βAnomer: (R)-{1-[3,5-bis-(trifluoromethyl)phenyl]ethyl} 2-O-p-fluorobenzyl-3,4-O-isopropylidene-6-O-tert-butyldiphenylsilyl-α-D-galactopyranoside, 26.

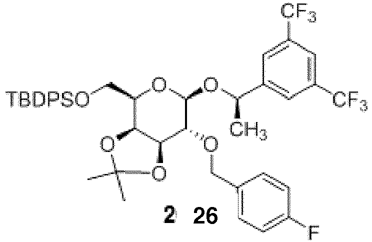

¹H-NMR (500MHz, CDCl₃): δ 7.85 (bs, 1 H), 7.79-7.74 (m, 3H), 7.66-7.61 (m, 4H), 7.43-7.32 (m, 7H), 7.05-7.00 (m, 2H), 4.97 (c, *J*=6.5 Hz, 1 H), 4.82 (s, 2H), 4.46 (d, *J*=8.0 Hz, 1H), 4.25 (dd, *J*=1.7 and 5.5 Hz, 1H), 4.17 (dd, *J*=5.8 and 6.7 Hz, 1H), 3.87 (dd, *J*=6.0 and 8.5 Hz, 1 H), 3.77-3.71 (m, 2H), 3.42 (t, *J*=7.5 Hz, 1 H), 1.50 (d, *J*=6.5 Hz, 3H), 1.36 (s, 3H), 1.33 (s, 3H), 1.02 (s, 9H). ¹³C NMR (500 MHz, CDCl₃) δ 162.6, (d, *J*_{CF}=245.2 Hz), 148.4, 146.3, 135.7, 135.6, 134.1, (d, *J*_{CF}=3.1 Hz), 133.5, 133.3, 131.9, (c, *J*_{CF}=33.1 Hz), 131.6, (c, *J*_{CF}=33.1 Hz), 129.9, (d, *J*_{CF}=8.2 Hz), 129.8, 127.8, 127.5, 125.8 (d, *J*_{CF}=2.2 Hz), 123.5 (c, *J*_{CF}=272.3 Hz), 121.4, 115.3 (d, *J*_{CF}=21.5 Hz), 110.1, 100.7, 79.7, 79.3, 74.9, 73.6, 73.3, 73.0, 62.6, 28.0, 26.8, 26.4, 25.8, 22.3, 19.3. HRMS: calculated for C₄₂H₄₅O₆F₇SiNa: [M+Na]⁺ 829.2771 found 829.2809 (4.5 ppm).

### Derivatives of L-arabinose 8 and 7:

The synthesis follows a procedure similar to that described for the preparation of galactose derivatives **25** and **26**, starting from **24** (4.85 g, 11.00 mmol), (1*R*)-1-[3,5-bis(trifluoromethyl)phenyl]ethanol (8.50 g, 33.00 mmol) and trimethylsilyl trifluoromethanesulfonate (0.14 mL, 0.77 mmol). A yellow oil is obtained with a mixture of the two α:β anomers with a ratio of 2:1. Purification by column chromatography using ethyl-hexane acetate with a ratio of 1:4 yields the β anomer (1.47 g, 2.75 mmol) as a minority product with a yield of 25% and the majority product is the α anomer (3.55 g, 6.60 mmol) with a yield of 60%.

### (R)-{1-3,5-bis-(trifluoromethyl)phenyl]ethyl} 2-O-p-fluorobenzyl-3,4-O-isopropylidene-β-L-arabinopyranoside, 8.

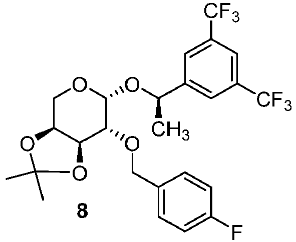

¹H-NMR (500MHz, CDCl₃): δ 8.04 (bs, 2H), 7.96 (bs, 1 H), 7.24-7.22 (m, 2H), 6.99-6.94 (m, 2H), 4.99 (c, *J*=6.6 Hz, 1H), 4.66 (d, *J*=3.3 Hz, 1H), 4.57 (bs, 2H), 4.37 (dd, *J*=5.7 and 7.8 Hz, 1H), 4.32 (dd, *J*=2.5 and 5.6 Hz, 1H), 4.11 (dd, *J*=2.9 and 13.4 Hz, 1H), 3.90 (d, *J*=13.4 Hz, 1H), 3.44 (dd, *J*=3.4 and 7.9 Hz, 1H), 1.51 (d, *J*=6.6 Hz, 3H), 1.34 (s, 3H), 1.32 (s, 3H). ¹³C NMR (500 MHz, CDCl3) δ 163.8, (d, *J*_{CF}=244.4 Hz), 148.0, 135.3 (d, *J*_{CF}=3.2 Hz), 132.9 (c, *J*_{CF}=32.9 Hz), 130.8 (d, *J*_{CF}=8.2 Hz), 128.2, 128.1, 124.9 (c, *J*_{CF}=272.2 Hz), 122.4 (c, *J*_{CF}=3.9 Hz), 115.9 (d, *J*_{CF}=21.3 Hz), 110.0, 96.4, 77.8, 76.6, 74.9, 72.2, 60.2, 28.4, 26.5, 24.5. [α]²⁰_{D}: +57.00 (c 1, chloroform).

### (R)-{1-3,5-bis-(trifluoromethyl)phenyl]ethyl} 2-O-p-fluorobenzyl-3,4-O-isopropylidene-α-L-arabinopyranoside, 7.

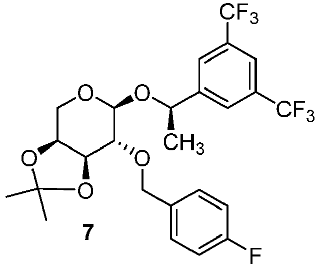

¹H-NMR (500MHz, CDCl₃): δ 7.95 (bs, 2H), 7.84 (bs, 1H), 7.44-7.40 (m, 2H), 7.08-7.03 (m, 2H), 5.03 (c, *J*=6.5 Hz, 1 H), 4.82 (bs, 2H), 4.61 (d, *J*=7.3 Hz, 1 H), 4.24-4.21 (m, 1 H), 4.17 (t, *J*=6.5 Hz, 1 H), 3.86 (dd, *J*=3.1 and 13.2 Hz, 1 H), 3.68 (dd, *J*=3.6 and 13.1 Hz, 1H), 3.47-3.43 (m, 1H), 1.53 (d, *J*=6.5 Hz, 3H) 1.36 (s, 3H), 1.32 (s, 3H). ¹³C NMR (500 MHz, CDCl₃) δ 163.8, (d, *J*_{CF}=243.0 Hz), 149.0, 135.7 (d, *J*_{CF}=3.2 Hz), 132.6 (c, *J*_{CF}=33.0 Hz), 131.1 (d, *J*_{CF}=7.9 Hz), 127.7 (2), 124.9 (c, *J*_{CF}=272.3 Hz), 121.9 (c, *J*_{CF}=3.9 Hz), 115.9 (d, *J*_{CF}=21.8 Hz), 111.0, 102.6, 81.0, 79.6, 76.8, 74.5, 73.6, 63.7, 28.1, 26.2, 23.0. [α]²⁰_{D}: +10.99 (c 1, chloroform).

### EXAMPLE 3. Selective deprotection of position 6.

### General procedure

Tetrabutyl ammonium fluoride 1 M (5 equiv.) is added to a solution of **25** or **26** (1 equiv.) in tetrahydrofuran and under an argon atmosphere. After stirring for 1 hour, an ether and saturated NaCl solution is added. The aqueous phase is extracted with ethyl acetate, the organic residues are dried over anhydrous sodium sulfate, and the solvent evaporated in vacuo. The residue obtained is purified by column chromatography, using ethyl acetate-hexane as the eluent with a ratio of 1:4.

### Alpha anomer: (R)-{1-[3,5-bis-(trifluoromethyl)phenyl]ethyl} 2-O-p-fluorobenzyl-3,4-O-isopropylidene-α-D-galactopyranose, 27.

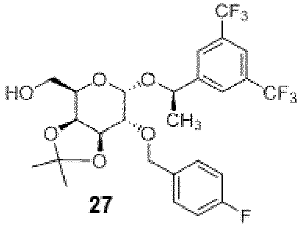

The synthesis follows the general procedure from **25** (0.70 g, 0.87 mmol) in THF (20 mL) and tetrabutyl ammonium fluoride 1M (4.34 mL, 4.34 mmol). This yields **27** (0.40 g 0.70 mmol) as a yellow syrup. Yield: 80%. ¹H-NMR (500MHz, CDCl₃): δ 7.91 (bs, 2H), 7.83 (bs, 1H), 7.20-7.17 (m, 2H), 6.97-6.92 (m, 2H), 4.92 (c, *J*=6.7 Hz, 1H), 4.64 (d, *J*=3.5 Hz, 1H), 4.60 (d, *J*=11.3 Hz, 2H), 4.47 (dd, *J*=5.6 and 7.9 Hz, 1H), 4.30 (dd, *J*=2.7 and 5.6 Hz, 1 H), 4.19-4.16 (m, 1 H), 3.97, (dd, *J*=6.0 and 11.8 Hz, 1 H), 3.87 (dd, *J*=3.9 and 11.8 Hz, 1 H), 3.42 (dd, *J*=3.6 and 8.0 Hz, 1 H), 1.52 (d, *J*=6.7 Hz, 3H), 1.35 (s, 3H), 1.33 (s, 3H). ¹³C NMR (500 MHz, CDCl₃) δ 162.4, (d, *J*_{CF}=244.2 Hz), 145.5, 133.5 (d, *J*_{CF}=3.1 Hz), 131.9, (c, *JCF*=*33.3 Hz), 129.5, (d, JCF*=*8.1 Hz), 126.7, 123.3, (c, JCF=272.8 Hz), 121.7, 115.1 (d, JCF=21.4 Hz), 109.5, 95.1, 76.1, 75.5, 74.5, 72.9, 71.3, 67.9,* HRMS: calculated for C₂₆H₂₆F₇O₆: [M]⁺ 568.1792 found 568.1790 (0.5 ppm).

### Beta anomer: (R)-{1-[3,5-bis-(trifluoromethyl)phenyl]ethyl} 2-O-p-fluorobenzyl-3,4-O-isopropylidene-β-D-galactopyranose, 28

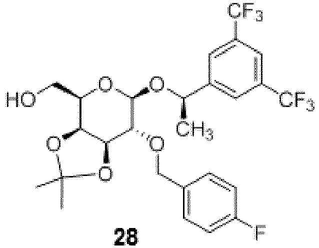

The synthesis follows the general procedure from **26** (0.70 g, 0.87 mmol) in THF (20 mL) and tetrabutyl ammonium fluoride 1 M (4.34 mL, 4.34 mmol). A yield of 0.37 g of **28** (0.65 mmol) is obtained as a yellow syrup. Yield: 75%. ¹H-NMR (500MHz, CDCl₃): δ 7.84 (bs, 2H), 7.79 (bs, 1H), 7.39-7.36 (m, 2H), 7.05-7.00 (m, 2H), 4.98 (c, *J*=6.4 Hz, 1H), 4.82 (bs, 2H), 4.49 (d, *J*=7.9 Hz, 1H), 4.19 (dd, *J*=5.8 and 6.7 Hz, 1H), 4.13-4.09 (m, 2H), 3.80-3.76 (m, 1H), 3.72-3.70, (m, 1H), 3.44 (dd, J=7.0 and 7.8 Hz, 1H), 1.54 (d, *J*=6.5 Hz, 3H), 1.37 (s, 3H), 1.32 (s, 3H). ¹³C NMR (500 MHz, CDCl₃) δ 162.6, (d, *J*_{CF}=245.6 Hz), 146.5, 134.0 (d, *J*_{CF}=3.0 Hz), 131.7, (c, *J*_{CF}=33.4 Hz), 129.9, (d, *J*_{CF}=8.2 Hz), 126.5, 123.5, (c, *J*_{CF}=273.0 Hz), 121.5, 115.3 (d, *J*_{CF}=21.5 Hz), 110.4, 101.5, 79.6, 79.4, 76.3, 73.9, 73.6, 73.0, 62.3, 27.8, 26.4, 22.8. HRMS: calculated for C₂₆H₂₆F₇O₆: [M]⁺ 568.1792 found 568.1789 (0.5 ppm).

### EXAMPLE 4. Deprotection of positions 3 and 4.

### General procedure

A catalytic quantity of CSA is added to a solution of 3,4-isopropylene derived **27**, **28**, **7 or 8** (1 equiv.) in methanol and left stirring overnight. After that time the solvent is evaporated in vacuo and the residue obtained is purified by filtration with silica using ethyl as the eluent.

### Galactose derivatives 4 and 3:

### Alpha anomer: (R)-{1-[3,4-bis-(trifluoromethyl)phenyl]ethyl} 2-O-p-fluorobenzyl-α-D-galactopyranose, 4

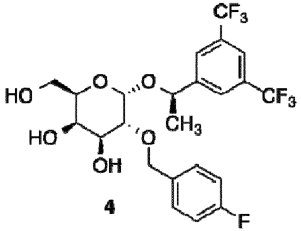

The synthesis follows the general procedure from **27** (0.50 g, 0.88 mmol) in methanol (20 mL). This yields **4** (0.46 g, 0.87 mmol) as a white solid. ¹H-NMR (500MHz, CDCl₃): δ 7.89 (bs, 2H), 7.84 (bs, 1H), 7.17-7.14 (m, 2H), 6.98-6.94 (m, 2H), 4.92 (c, *J*=6.5 Hz, 1H), 4.79 (d, *J*=3.5 Hz, 1H), 4.53 (d, *J*=11.9 Hz, 1H), 4.34 (d, *J*=11.8 Hz, 1H) 4.16-4.10 (m, 2H), 4.02-3.88 (m, 3H), 3.69 (dd, *J*=3.5 and 9.8 Hz, 1H), 2.79 (bs, 1H), 2.34 (d, *J*=2.6 Hz, 1 H), 2.30 (dd, *J*=3.6 and 7.1 Hz, 1 H), 3.07 (d, *J*=6.7 Hz, 3H). ¹³C NMR (500 MHz, CDCl₃) δ 162.7, (d, *J*_{CF}=244.0 Hz), 145.8, 133.4 (d, *J*_{CF}=3.5 Hz), 132.2, (c, *J*_{CF}=33.4 Hz), 129.8, (d, *J*_{CF}=7.9 Hz), 126.8 (2), 123.4, (c, *J*_{CF}=271.8 Hz), 122.0 (2), 115.7 (d, *J*_{CF}=21.0 Hz), 95.1, 75.9, 73.3, 72.1, 70.8, 69.9, 69.1, 63.5, 24.4. HRMS: calculated for C₂₃H₂₃F₇O₆: [M+Na]⁺ 551.1281 found 551.1264 (-3.0 ppm).

### Beta anomer: (R)-{1-[3,4-bis-(trifluoromethyl)phenyl]ethyl} 2-O-p-fluorobenzyl-β-D-galactopyranose, 3

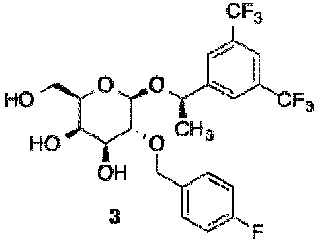

The synthesis follows the general procedure from **28** (0.50 g, 0.88 mmol) in methanol (20 mL). This yields **3** (0.45 g, 0.85 mmol) as a white solid. Yield: quantitative. ¹H-NMR (500MHz, CDCl₃): δ 7.81 (bs, 2H), 7.79 (bs, 1 H), 7.36-7.33 (m, 2H), 7.07-7.02 (m, 2H), 4.99 (c, *J*=6.4 Hz, 1 H), 4.88 (d, *J*=11.5 Hz, 1 H), 4.71 (d, *J*=11.5 Hz, 1 H), 4.59 (d, *J*=6.2 Hz, 1 H), 3.91 (dd, *J*=3.6 and 5.9 Hz, 1 H), 3.77 (dd, *J*=3.9 and 12.6 Hz, 1 H), 3.72 (dd, *J*=3.4 and 8.0 Hz, 2H), 3.58 (dd, *J*=6.3 and 8.0 Hz, 1 H), 3.46 (dd, *J*=2.0 and 12.8 Hz, 1 H), 2.70 (bs, 1 H), 2.49 (bs, 1 H), 1.53 (d, *J*=6.4 Hz, 3H). ¹³C NMR (500 MHz, CDCl₃) δ 162.7, (d, *J*_{CF}=247.0 Hz), 146.0, 134.0 (d, *J*_{CF}=2.9 Hz), 131.8, (c, *JCF*=*33.4 Hz), 129.8, (d, JCF=8.2 Hz), 126.4, 123.0, (c, JCF=272.7 Hz), 121.7, 115.6 (d, JCF*=*21.2 Hz), 101.0, 78.6, 75.2, 73.7, 72.1, 67.4, 64.7, 22.1.* HRMS: calculated for C₂₃H₂₃F₇O₆: [M+Na]⁺ 551.1281 found 551.1262 (-3.0 ppm).

### Arabinose derivatives 6 and 5:

### (R)-{1-[3,4-bis-(trifluoromethyl)phenyl]ethyl} 2-O-p-fluorobenzyl-β-L-arabinopyranoside, 6.

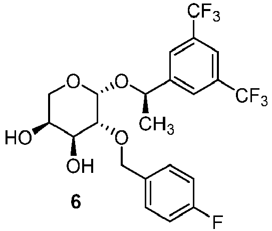

The synthesis follows the general procedure from **8** (1.47 g, 2.75 mmol) in methanol (60 mL). The procedure yields **6** (1.35 g, 2.72 mmol) as a brown oil with a quantitative yield. ¹H-NMR (300MHz, CDCl₃): δ 7.89 (bs, 2H), 7.83 (bs, 1H), 7.19-7.15 (m, 2H), 7.00-6.94 (m, 2H), 4.92 (c, *J*=6.6 Hz, 1H), 4.73 (d, *J*=3.3 Hz, 1H), 4.53 (d, *J*=11.7 Hz, 1H), 4.32 (d, *J*=11.9 Hz, 1H), 4.16-4.09 (m, 1H), 3.95-3.91 (m, 1H), 3.78 (dd, *J*=1.8 and 12.4 Hz, 1H), 3.67 (dd, *J*=3.4 and 9.5 Hz, 1H) 1.54 (d, *J*=6.6 Hz, 3H). [α]²⁰_{D}: +12.02 (c 1, chloroform).

### (R)-{1-[3,4-bis-(trifluoromethyl)phenyl]ethyl} 2-O-p-fluorobenzyl-α-L-arabinopyranoside, 5.

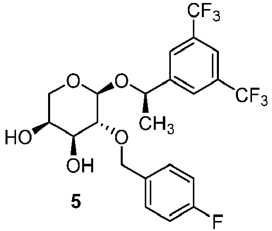

The synthesis follows the general procedure from **7** (3.55 g, 6.60 mmol) in methanol (160 mL). The procedure yields **5** (3.36 g, 6.55 mmol) as an orange oil with a quantitative yield. ¹H-NMR (500MHz, CDCl₃): δ 7.81 (bs, 2H), 7.79 (bs, 1H), 7.36-7.32 (m, 2H), 7.06-7.02 (m, 2H), 4.98 (c, *J*=6.5 Hz, 1H), 4.88 (d, *J*=11.4 Hz, 1H), 4.71 (d, *J*=11.6 Hz, 1 H), 4.58 (d, *J*=6.3 Hz, 1 H), 3.77 (dd, *J*=3.9 and 12.6 Hz, 1 H), 3.71 (dd, *J*=3.6 and 8.1 Hz, 1 H) 3.58 (dd, *J*=6.4 and 8.0 Hz, 1 H), 3.47-3.43 (m, 2H), 1.53 (d, *J*=6.5 Hz, 3H). ¹³C NMR (500 MHz, CDCl₃) δ 162.6, (d, *J*_{CF}=246.7 Hz), 146.1, 134.0 (d, *J*_{CF}=3.1 Hz), 131.8 (c, *J*_{CF}=33.2 Hz), 129.8 (d, *J*_{CF}=8.1 Hz), 126.4, 123.4 (c, *J*_{CF}=274.8 Hz), 121.6, 115.6 (c, *J*_{CF}=21.3 Hz), 101.1, 78.7, 75.2, 73.8, 72.1, 67.5, 64.8, 22.1. [α]²⁰_{D}: +8.07 (c 1, chloroform).

### EXAMPLE 5.

Dimethoxymethyl benzene (1.1 equiv.) and a catalytic amount of CSA are added to a solution of **3** or **4** (1 equiv.) in dimethylformamide (DMF). The reaction is left to rotate in a rotary evaporator at 40 °C for 1 hour. After that time, it is neutralised with saturated NaHCO₃ solution, the aqueous phase is extracted with dichloromethane, the organic extracts are dried over anhydrous sodium sulfate and the solvent evaporated in vacuo. The residue obtained is purified by column chromatography using ethyl acetate-hexane in a ratio of 1:4.

### Alpha anomer: (R)-{1-[3,5-bis-(trifluoromethyl)phenyl]ethyl} 2-O-p-fluorobenzyl-(R)-[4,5-O-benzylidene]-α-D-galactopyranoside, 2

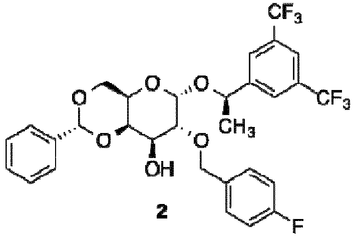

The synthesis follows the general procedure from **4** (0.10 g, 0.19 mmol) in DMF (15 mL) and dimethoxymethyl benzene (0.32 mL, 0.21 mmol). The procedure yields 2 (0.11 g, 0.18 mmol) as a white solid. Yield: 95%. P.f.: 158-159 °C. ¹H-NMR (500MHz, CDCl₃): δ 7.90 (bs, 2H), 7.84 (bs, 1H), 7.47-7.43 (m, 2H), 7.39-7.34 (m, 3H), 7.16-7.13 (m, 2H), 6.95-6.90 (m, 2H), 4.93 (c, *J*=6.6 Hz, 1 H), 4.80 (d, *J*=3.5 Hz, 1 H), 4.52 (d, *J*=2.5 Hz, 2H), 4.36-4.35 (m, 1H), 4.32 (dd, *J*=1.4 and 12.6 Hz, 1H), 4.28-4.23 (m, 1H), 4.14 (dd, *J*=1.8 and 12.7 Hz, 1H), 3.84 (bs, 1H), 3.75 (dd, *J*=3.6 and 10.0 Hz, 1H), 2.33 (d, *J*=8.8 Hz, 1 H), 1.53 (d, *J*=6.6 Hz, 3H). ¹³C NMR (500 MHz, CDCl₃) δ 162.6, (d, *J*_{CF}=246.1 Hz), 145.6, 137.5, 133.6 (d, *J*_{CF}=3.0 Hz), 132.1, (c, *JCF*=*33.6 Hz), 129.7, (d, JCF*=*8.2 Hz), 129.4, 128.4, 126.9, 126.4, 123.4, (c, JCF*=*272.1 Hz), 122.0, 115.4 (d, JCF*=*21.8 Hz), 101.5, 96.0, 76.3, 76.1, 73.3, 72.6, 69.4, 68.8, 63.3, 24.3.* HRMS: calculated for C₃₀H₂₇O₆NaF₇: [M+Na]⁺ 639.1594 found 639.1565 (-4.5 ppm).

### Beta anomer: (R)-{1-[3,5-bis-(trifluoromethyl)phenyl]ethyl} 2-O-p-fluorobenzyl-(R)-[4,5-O-benzylidene]-β-D-D-galactopyranoside, 1

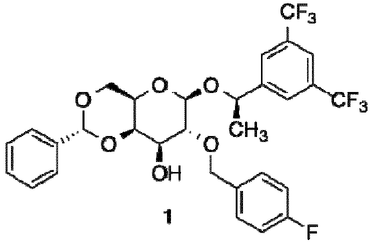

The synthesis follows the general procedure from **3** (0.10 g, 0.19 mmol) in DMF (15 mL) and dimethoxymethyl benzene (0.32 mL, 0.21 mmol). The procedure yields **1** (0.10 g, 0.17 mmol) as a white solid. Yield: 92%. P.f.: 131 °C. 1H-N^{M}R (500MHz, CDCl3): δ 7.90 (bs, 2H), 7.80 (bs, 1 H), 7.49-7.47 (m, 2H), 7.39-7.36 (m, 5H), 7.06-7.01 (m, 2H), 5.03 (c, *J*=6.6 Hz, 1 H), 4.93 (d, *J*=11.3 Hz, 1 H), 4.79 (d, *J*=11.2 Hz, 1 H), 4.57 (d, *J*=7.6 Hz, 1H), 4.19 (dd, *J*=1.0 and 3.9 Hz, 1H), 4.08 (dd, *J*=1.4 and 12.5 Hz, 1H), 3.98 (dd, *J*=1.9 and 12.5 Hz, 1H), 3.74 (td, *J*=3.8 and 8.9 Hz, 1H), 3.67-3.64 (m, 1H), 3.57-3.35 (m, 1 H), 2.45 (d, *J*=8.4 Hz, 1 H), 1.56 (d, *J*=6.6 Hz, 3H). ¹³C NMR (500 MHz, CDCl₃) δ 162.6, (d, *J*_{CF}=246.6 Hz), 146.6, 137.7, 134.4 (d, *J*_{CF}=2.8 Hz), 131.6, (c, *J*_{CF}=33.4 Hz), 129.7, (d, *J*_{CF}=8.1 Hz), 129.4, 128.4, 126.7, 126.6, 123.6, (c, *J*_{CF}=272.7 Hz), 121.4, 115.4 (d, *J*_{CF}=21.5 Hz), 101.9, 101.6, 79.8, 76.1, 75.5, 74.6, 73.0, 69.0, 66.7, 22.8. HRMS: calculated for C₃₀H₂₇O₆NaF₇: [M+Na]⁺ 639.1594 found 639.1563 (-4.3 ppm).

### EXAMPLE 6. Antagonistic activity of the NK1 receptors of the synthesised carbohydrate derivatives.

The capacity of the synthesised carbohydrate derivatives to modulate the NK1 receptor has been determined experimentally through the Ip-one assay and affinity studies through the displacement of marked SP ([¹²⁵I]BH-SP).

### 6.1. Results obtained.

**6.1.1. IP-One assay results:** The results of the NK1 receptor antagonist activity study are shown as an example, the results are for the IP-One assay on synthesised carbohydrate derivatives **2**, **3**, **4**, **5** and **6** along with the commercial antagonists L-732,138 and CP-96345 (the first non-peptide NK1 antagonist), which have been used as a reference. SP was used as a positive control in the test.

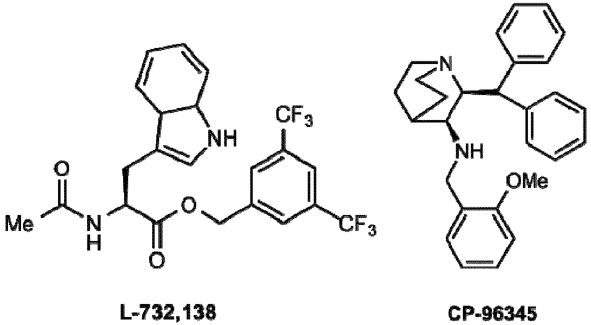

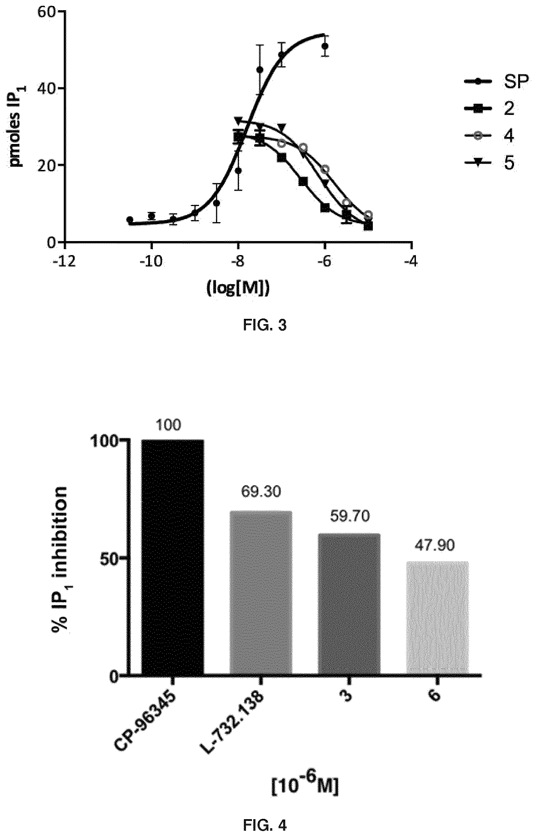

All the compounds tested antagonise SP through the NK1 receptor. We highlight the results for derivatives **2**, **4** and **5**, which are excellent inhibitors of SP activity, with very similar *K_{inact}* values to those of the reference antagonist (FIG. 2 and 3):
*K_{act}* **SP:** 1.64E-08 ± 0.62 M; *K_{inact}* **L-732,138:** 8.37E-08 ± 2.63 M; *K_{inact}* **CP-96345:** 3.65E-09 ± 0.55 M; *K_{inact}* **2:** 2.37E-07 ± 1.16 M; *K_{inact}* **4:** 7.37E-07 ± 2.71 M; *K_{inact}* **5**: 5.60E-07 ± 0.53 M

Of all the derivatives, derivative **2** showed the greatest antagonist activity.
The inhibition percentages were obtained at a ligand concentration of 10⁻⁶M (FIG. 4):
**L-732,138:** 69% SP inhibition; Derivative **3:** 60% SP inhibition; Derivative **6:** 48% SP inhibition.

**6.1.2. Results of the marked SP displacement study.** The affinity of synthesised carbohydrate derivatives **2**, **4**, **5** and **8** are shown as an example.

Summary of the affinity results:

| Compound | Concentration (M) | % Inhibition of the control specific affinity |
|---|---|---|
| **2** | 6.0E-05 | ≥100 |
| **4** | 6.0E-05 | ≥100 |
| **5** | 6.0E-05 | ≥100 |
| **8** | 6.0E-05 | ≥100 |

The compounds show have a greater affinity for the NK1 receptors than SP.

### 6.2. General procedure for determining the antagonist activity of NK1 receptors.

### 6.2.1. Ip-one assay:

**Cell culture and transfection.** The cell lines were obtained from the American Type Culture Collection (Manassas, Virginia, USA). The culture media, foetal bovine serum (FBS) and additives were supplied by Invitrogen.

The CHO cells were cultured on a *Dulbecco's modified Eagle's* medium with a 10% FBS supplement, penicillin/streptomycin, 100U/ml and L-glutamine 2 mM at 37°C in a humid atmosphere of 95% air and 5% CO₂. Non-essential amino acids were also added to the medium (Invitrogen).

Transient transfection of the cell lines was carried out by electroporation in a volume of 300 µL with a total of 10 µg DNA (pRK5 Neo-NK1 wild type) plasmid (using up to 500 ng of pRK5como carrier DNA to achieve 10 µg) and with 10⁷ cells in the electroporation buffer (50 mM K₂HPO₄, 20 mM CH₃COOK, 20 mM KOH and 26 mM MgSO₄, pH 7.4). After electroporation (280 V, 1mF, GeneZapper 450/2500; IBI, New Haven, Connecticut, USA), the cells were suspended in a complete medium and seeded on well culture plates with a density of 10⁵ per well. The 96-well culture plates were first covered with polyornithine diluted in PBS, then incubated at 37°C for 30 minutes and they were then washed with PBS before being seeded.

**ELISA.** The cells were transfected with pRK5-NK1-6His to measure the expression of the transfected receptors. The cells were fixed 24 hours after electroporation using paraformaldehyde at 4% in PBS for 5 minutes and washed 3 times with PBS. A 30-minute block was carried out with PBS and 1% unsupplemented FBS before incubation with the anti-6 His primary antibody (0.5 µg/ml) for 30 minutes. The cells were then washed three times with PBS + 1% FBS for 5 minutes and incubated for 30 minutes with a horseradish peroxidase conjugated anti-mouse antibody (1/1000; Amersham, Orsay,France). The cells were then washed three times with PBS + 1% FBS and three times with PBS. Then 60 µl of PBS and 20 µl of Supersignal ELISA Femto (Perbio-Pierce, Brebières, France) were added to the wells. The luminescence was read using a Wallac Victor2 (PerkinElmer Life and Analytical Sciences, Courtaboeuf, France).

**Second Messenger accumulation (IP₁).** The activation and inhibition of the route of the IP by the NK1 receptor agonists and antagonists, respectively, was determined using the dynamic IP-One kit (Cisbio Bioassays, Bagnols-sur-Cèze, France). After transfection, 10⁵ cells were distributed in 100 µl of a complete medium into a 96-well assay plate (Greiner Bio-One, Courtaboeuf, France). After 24 hours, the medium was eliminated and replaced by 40 µl of an incubation medium that contained the agonist and/or antagonist in an appropriate concentration. The homogeneous time-resolved fluorescence-fluorescence resonance energy transfer (HTRF-FRET) assay was performed as indicated in the literature (Maurel, D.; Kniazeff, J.; Mathis, G.; Tinquet, E.; Pin, J.P.; Ansanay, H. Anal Biochem. 2004, 329, 253). the inhibitory effect of the non-peptidic NK1 antagonist in SP-induced IP₁ accumulation was studied in the same way as Arunlakshana and Schild (1959) (Arunlakshana, or.;) Schild, H.O. Br J Pharmacol. 1959, 14, 48). The 10 minute preincubation was followed by a 30 minute incubation with the antagonist and SP.

**Statistical analysis.** The statistical significance of the differences between the experimental groups was determined by *one-way or two-way* variance analysis followed by a post-hoc Duncan multiple-rank test to perform pairwise comparisons between the means. The Student *t* test was also used.

### 6.2.2. Displacement of marked Substance P:

A marked SP concentration of 0.15 nM was used for the displacement studies and the concentration of the products of the invention was 10⁻⁶ M. The incubation time was 60 minutes at room temperature and the reference ligand used was [Sar⁹, Met(O₂)¹¹]-SP with IC₅₀=0.29 nM (Heuillet, E. J. Neurochem. 1993, 60, 868-876).

### EXAMPLE 7. Selective anticancer activity of the new carbohydrate derivative NK1 antagonists.

### 7.1. Results obtained.

By way of example, the results are shown for the anticancer activity in lung cancer studies for derivatives **1**, **2**, **5**, **7**, **8**, **9** and **10.** Two commercial NK1 antagonists, **CP-96345** and **Aprepitant**, were used as a reference. The reference drug **cisplatin**, used in therapy to treat lung cancer, was used as a positive control.

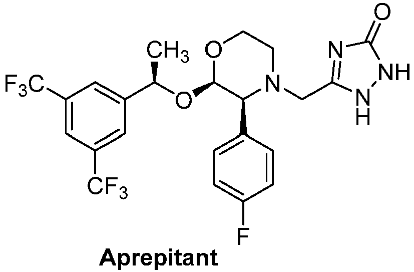

The IC₅₀ was determined in all cases through the MTT test, both for cancerous lung cells (A549) as well as in healthy lung cells (MRC-5) to determine the selectivity of the compounds. The following table shows the IC₅₀ values for the MRC-5 and A549 cell lines corresponding to cisplatin.

| | **IC₅₀ ± SEM (µM)** | | |
|---|---|---|---|
| **Compuesto** | **MCF7** | **MCF10** | **p** |
| **2** | 23.91±4.99 | 291.32±52.56 | 0.04057 |
| | **UACC-62** | **VH-10** | **p** |
| | 31.93±6.01 | 117.90±12.31 | 0.01692 |

The following table shows the IC₅₀ values for the cell lines MRC-5 and A549 corresponding to the commercial antagonists Aprepitant and CP-96345 and the new synthesised NK1 receptor antagonists.

| | **IC₅₀ ± SEM (µM)** | | |
|---|---|---|---|
| **Compounds** | **MRC-5** | **A549** | **p** |
| **1** | 141.16±1.82 | 28.00±6.85 | 0.0047 |
| **2** | 120.10±3.45 | 19.34±7.93 | 0.0046 |
| **5** | 50.41±3.27 | 20.82±4.02 | 0.0061 |
| **7** | - | 59.32±11.04 | - |
| **8** | 500.99±62.03 | 133.52±35.18 | 0.0159 |
| **9+10(1:1)** | 192.18±27.85 | 42.88±9.19 | 0.0082 |
| **Aprepitant** | 22.08±6.92 | 15.66±3.46 | >0.05 |
| **Cup-96345** | 57.90±8.07 | 46.83±8.89 | 0.4100 |

All of our compounds not only showed a clear anticancer activity, they were also selective for lung cancer cells, unlike the commercial NK-1 antagonists Aprepitant and CP-96345. Among all of them, we would highlight the derivative 7, which had a selectivity far superior to the others. All the derivatives except derivative **8** showed greater activity against the cancer line than Aprepitant and CP-96345. The most active derivatives were **1**, **2** and **5.**

According to these results, the activity and selectivity of our derivative **2** matches those of cisplatin. Anticancer activity studies were, therefore, carried out with ligand **2** on other cell lines.

The possible anticancer activity of compound **2** was evaluated in a total of three in-vitro cancer models: breast cancer, lung cancer and melanoma. In all three models, this compound was shown to be more cytotoxic to cancer cells than to normal cells. This selectivity was observed from the lowest concentrations tested and over a range of concentrations, we can, therefore, conclude that this compound presents selective cytotoxic activity. Specifically, it has proved to be 4, 6, and 12 times more selective for melanoma, lung and breast cancer cells respectively, with respect to the corresponding normal cells.

The following table shows the IC₅₀ values for cell line MCF7 (human breast adenocarcinoma cells) and MCF10 (non-malignant human breast cells) as well as UACC-62 (human melanoma cells) and VH-10 lines (non-malignant human skin cells) for compound **2.**

| | **IC₅₀ ± SEM (µM)** | | |
|---|---|---|---|
| **Compound** | **MCF7** | **MCF10** | **p** |
| **2** | 23.91±4.99 | 291.32±52.56 | 0.04057 |
| | **UACC-62** | **VH-10** | **p** |
| | 31.93±6.01 | 117.90±12.31 | 0.01692 |

### 7.2. General procedure for the determination of selective anticancer activity for the new NK1 receptor antagonists.

**Cell lines.** A549 human cells from lung adenocarcinoma, MRC5 non-malignant human lung cells and UACC-62 human melanoma cells were obtained from the European Collection of Authenticated Cell Cultures (ECACC). MCF7 human breast adenocarcinoma cells and MCF10 human non-malignant breast cells were generously provided by Dr Ruano and Dr Daza (University of Seville, Spain). VH-10 non-malignant human skin cells were generously provided by Dr Helleday (Karolinska Institute, Sweden).
All the culture media used were supplemented with 2 mM glutamine, 50 µg/ml penicillin, 50 µg/ml streptomycin and 10% fetal bovine serum. An exception was the medium used for the cultivation of MCF10 cells which was a 1:1 mixture of DMEM and HAM's F12 media supplemented with 2 mM glutamine, 50 µg/ml penicillin, 50 µg/ml streptomycin, 20 ng/ml growth factors, 100 ng/ml cholera toxin, 10 µg/ml insulin, 500 ng/ml hydrocortisone and 5% horse serum.

All the cell lines were cultured at 37 °C, 5% CO₂ and controlled humidity in an incubator.

All the products used for the cell culture were obtained from the PAA laboratory, with the exception of the media for the MCF10 cell line which was provided by Dr Navarro.

**Cell viability assay.** The cells were exposed to the compounds for 48 hours. After the treatment, the media were withdrawn and cells were incubated for 3-5 hours with 125 µl MTT (1 mg/ml MTT in culture medium). Then, 80 µl 20% SDS in 0.02 M HCl was added to solubilise the formazan-MTT crystals and the plates were incubated overnight in an incubator at 37 °C and 5% CO₂. Finally, the plates were read using a plate reader spectrophotometer at 540 nm. The absorbance was directly proportional to cell viability, which is expressed as a percentage relative to the control. All the results were obtained from at least three independent experiments and expressed as mean ± the standard error of the mean (SEM).

## Claims

1. Compound of general formula **I**, 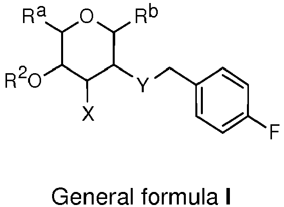 or any of its stereoisomers, or a pharmaceutically acceptable salt thereof, wherein:
- R^{a} is selected from H and CH₂OR¹, and wherein R¹ is selected from
hydrogen,
a C₁-C₂₀ alkyl group,
a C₆-C₂₀ aryl group,
a COR^{1a} group, wherein R^{1a} is independently selected from methyl, *tert-*butyl, and phenyl,
a group which together with R² forms a cyclic chain, and
a SiR'R"R''' group, wherein R', R" and R''' are independently selected from methyl, ethyl, *tert*-butyl and phenyl;
R^{b} independently selected from a C₁-C₂₀ alkyl group, a C₆-C₂₀ aryl group, OH and a group of formula II, wherein Z is selected from O and S and the carbon adjacent to Z is chiral and has configuration R or S; 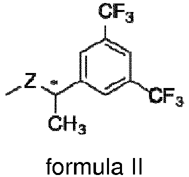
- X is selected from OR³, NR⁴R⁵ and a 3 to 15 members heterocyclic chain, wherein:
• R³ is selected from H, a C₁-C₂₀ alkyl group, a C₆-C₂₀ aryl group, an acyl group, a alkylsulfonyl group, an arylsulfonyl group, a group of formula III, a group of formula IV, a group of formula V wherein R'''' is a C₁-C₂₀ alkyl group or a C₆-C₂₀ aryl group, and a group that forms a cyclic chain with R²;
• R⁴ is selected from H, a C₁-C₂₀ alkyl group and a C₆-C₂₀ aryl group;
• R⁵ is selected from H, a group of formula III, a group of formula IV and a group of formula V wherein R"" is a C₁-C₂₀ alkyl group or a C₆-C₂₀ aryl group; 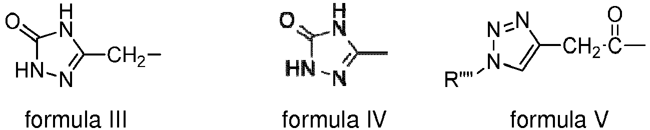
- Y is selected from O, S and NH; and
- R² is H, or forms a cyclic chain with 5 or 6 members along with R¹ or R³, in such a way that in said cyclic chain the O adjacent to R² is separated from the O adjacent to R¹ or R³ by at least one C atom consisting of -C(R⁶)(R⁷)-, and wherein R⁶ and R⁷ are independently selected from H, a C₁-C₂₀ alkyl group and a C₆-C₂₀ aryl group.

2. Compound according to claim 1, wherein R^{b} is the group of formula II in which Z is selected from O and S.

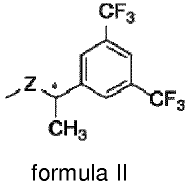

3. Compound according to claim 2, wherein Z is O.

4. Compound according to any of claims 1 to 3, wherein Y is O.

5. Compound according to any of claims 1 to 4, wherein X is OR³, and R³ is defined as described in claim 1.

6. Compound according to any of claims 1 to 5, wherein R^{a} is CH₂OR¹ and R² forms a cyclic chain along with R¹, wherein the O atom adjacent to R² is separated from the O atom adjacent to R¹ by a -C(R⁶)(R⁷)- group, such that R² forms a cyclic chain with 6 members along with R¹, wherein R⁶ and R⁷ are independently selected from H, a C₁-C₂₀ alkyl group and a C₆-C₂₀ aryl group.

7. Compound according to any of claims 1 to 6 wherein R^{a} is CH₂OR¹, R¹ and R² form a cyclic chain, and X is OH.

8. Compound according to any of claims 1 to 5, wherein X is OR³ and R² form a cyclic chain along with R³, wherein the O atom adjacent to R² is separated from the O atom adjacent to R³ by a -C(R⁶)(R⁷)- group, such that R² forms a cyclic chain with 5 members along with R³, and wherein R⁶ and R⁷ are independently selected from H, a C₁-C₂₀ alkyl group and a C₆-C₂₀ aryl group.

9. Compound according to any of claims 1 to 5 or 8, wherein X is OR³, R³ and R² form a cyclic chain, and R^{a} is H.

10. Compound according to any of claims 1 to 5, wherein X is OH and R² is H.

11. Compound according to claim 10, wherein R^{a} is selected from H and CH₂OH.

12. Compound according to any of claims 1 to 11 which is selected from the group consisting of:

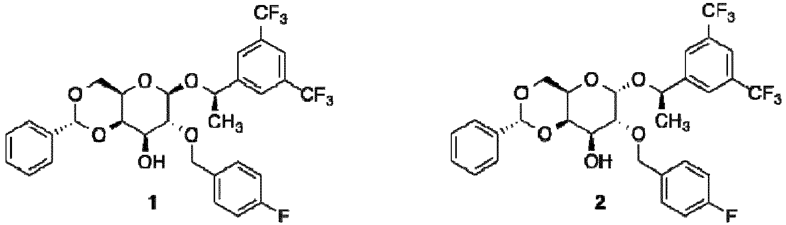

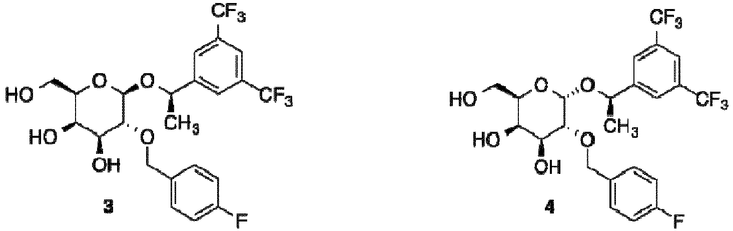

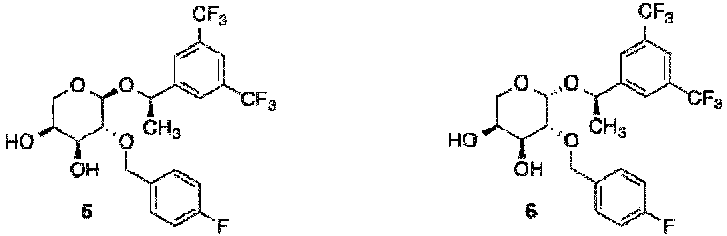

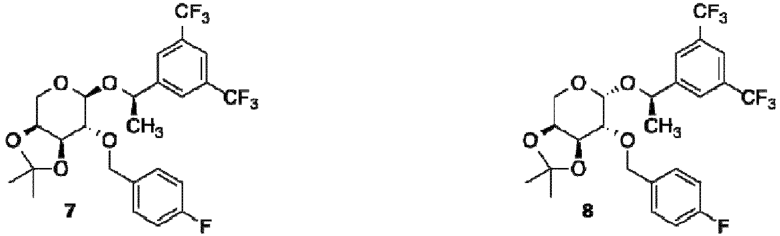

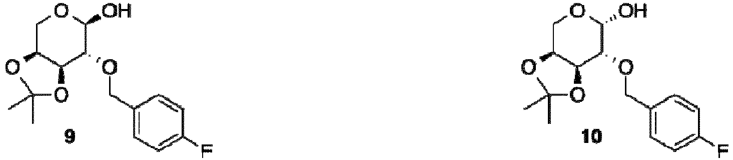

13. Pharmaceutical composition **characterised in that** it comprises at least one compound defined in any of claims 1 to 12, or a pharmaceutically acceptable salt thereof.

14. Use of at least one compound according to any of claims 1 to 12, or of a pharmaceutically acceptable salt thereof, for the manufacture of a pharmaceutical composition.

15. Use of at least one compound according to any of claims 1 to 12, or of a pharmaceutically acceptable salt thereof, for the manufacture of a pharmaceutical composition for the treatment and/or prevention of a disease.

16. Use according to claim 15, wherein the disease is selected from the group consisting of: a disorder of the central nervous system selected from Parkinson's disease, anxiety and depression, rheumatoid arthritis, asthma, inflammatory bowel disease, post-operative abdominal adhesion, migraine, inflammation, a chronic pulmonary disease selected from bronchial asthma or chronic obstructive lung disease (COPD), obstructive sleep apnea, a deregulation of cardiac function, arterial thrombosis, osteoporosis, obesity, resistance to insulin, Crohn's disease, emesis, and a cancer selected from melanoma, neuroblastoma, glioma, Hodgkin's lymphoma, lymphoblastic leukemia, rhabdomyosarcoma, Burkitt's lymphoma, lung carcinoma, Edwing's sarcoma, osteosarcoma, malignant ganglioma, and breast cancer.

17. Use according to claim 16, wherein the disease is selected from the group consisting of: melanoma, lung carcinoma and breast cancer.

18. Method for obtaining a compound of formula I as defined in any of claims 1 to 12, **characterised in that** it comprises at least the following steps:
a. Obtaining a thioglycoside of formula VIII, wherein Ra is defined as in any of claims 1, 6 or 7, R is selected from a C₆-C₂₀ aryl group and a C₁-C₂₀ alkyl group, X' is selected from an OH group and a N₃ group, and Y is selected from an OH, S and NH group; 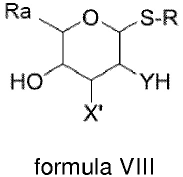
b. Reacting the thioglycoside of formula VIII with alcohol protecting reagents to obtain the compound with formula IX; 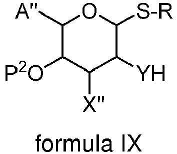 wherein:
- A" is selected from H and CH₂OP¹, wherein P¹ is selected from
a C₁-C₂₀ alkyl group,
a C₆-C₂₀ aryl group,
a COP^{1a} group, where R^{1a} is independently selected from methyl, *tert*-butyl and phenyl,
a group that forms a cyclic chain along with P², and
a SiR'R"R''' group, where R', R" and R''' are independently selected from methyl, ethyl, *tert*-butyl and phenyl,
- R is selected from a C₆-C₂₀ aryl group and a C₁-C₂₀ alkyl group,
- X" is selected from an N₃ group and an OP³ group, wherein P³ is a group that forms a cyclic chain with 5 members with P²,
- Y is selected from O, S and NH,
- P² is selected from
a SiR'R"R''' group wherein R', R" and R''' are independently selected from methyl, ethyl, *tert*-butyl and phenyl,
a group that forms a cyclic chain with 6 members with P1, and
a group that forms a cyclic chain with 5 members with P3,
so that in the cyclic chain the O adjacent to P² is separated from the O adjacent to P¹ or P³ by a C atom consisting of -C(R⁶)(R⁷)-, and where R⁶ and R⁷ are independently selected from H, a C₁-C₂₀ alkyl group and a C₆-C₂₀ aryl group;
c. Reacting the compound of formula IX with an halide or tosylate of p-fluorobenzyl to obtain the compound of formula X, wherein A", R, X" and P² are as previously defined, and Y is selected from O, S and NH; 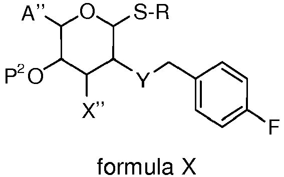
d. Transformation of the compound X obtained in the previous stage into a glycosyl donor compound of formula XI, wherein W is selected from OH, an SOR sulfoxide group, an O(OR)₂ phosphite group and a trichloracetimidate group; and 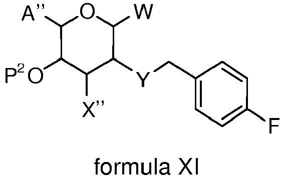
e. Transformation of the compound XI obtained in the previous stage into a compound of general formula I as described in any of claims 1 to 12, through a glycosidation reaction.

19. Method according to claim 18, **characterised in that** when R^{b} in the compound of general formula I is the group of formula II, said procedure comprises reacting the compound of formula XI with 2,2,2-trichloroacetonitrile in the presence of catalytic quantities of 1,8-diazabicyclo [5.4.0]undec-7-eno (DBU); and subsequently reacting the product obtained with 1-[3,5-bis(trifluoromethyl)phenyl]ethanol and trimethylsilyl trifluoromethanesulfonate.

20. Method according to claim 19, additionally comprising the selective protection and/or deprotection of groups in position 3, 4, 5 and/or 6 of the tetrahydropyranyl ring.

21. Method according to claim 19, additionally comprising the reduction and/or alkylation, acylation or Huisgen 1,3-dipolar reaction to obtain compounds of formula I wherein X is NR⁴R⁵ or a heterocyclic chain.

22. Method according to any of claims 18 to 21, additionally comprising at least one of the following steps:
f. Reacting the compound obtained in steps e with an azide-reducing agent, when in the first step of the procedure a compound of formula VIII is obtained wherein X' is a N₃ group;
g. Reacting the compound obtained in step e or f with tetrabutyl ammonium fluoride in THF;
h. Reacting the compound obtained in either of steps e, f or g with a catalytic quantity of 10-camphorsulfonic acid in methanol;
i. Reacting the compound obtained in any of the steps g or h with a dimethoxymethyl derivative with the formula CH₃O-C(R⁶)(R⁷)-OCH₃, wherein R⁶ and R⁷ are as defined in claim 1, if the first step of the procedure yields a compound of formula VIII wherein Ra is CH₂OH;
j. Reacting the compound obtained in any of steps f, g or h, with an alkyl halide of formula R⁸-X or an acyl halide of formula R⁹-CO-X, wherein R⁸ is a C₁-C₂₀ alkyl group, a C₆-C₂₀ aryl group or a group of formula III or formula IV as defined in claim 1, and wherein R⁹ is a group of formula V as defined in claim 1.
